# EUROPEAN PATENT SPECIFICATION

(11) **EP 0 376 218 B1**
(45) Date of publication and mention of the grant of the patent: **24.02.1999**
(21) Application number: 89123841.2
(22) Date of filing: 22.12.1989
(51) Int. Cl.: C07K 1/04, C07K 17/02, C07C 229/02

(54) **Racemization free attachment of amino acids to solid phase**
Racemisierungsfreies Verfahren zum Aufhängen von Aminosäuren auf einer Festphase
Rattachement sans racémisation des acides aminés aux phases solides

(30) Priority: 27.12.1988 US 290433
(43) Date of publication of application: 04.07.1990
(73) Proprietor: PERSEPTIVE BIOSYSTEMS, INC., Framingham, MA 01710 (US)
(72) Inventor: Bernatowicz, Michael S., Cambridge, MA 02139 (US)
(74) Representative: Henkel, Feiler, Hänzel

(56) References cited:
- FR-A- 2 286 811
- CHEMICAL ABSTRACTS, vol. 109, no. 5, 1st August 1988, page 658, abstract no. 38219k, Columbus, Ohio, US; F. ALBERICIO et al.: "Mild, orthogonal solid-phase peptide syntheis: use of N-dithiasuccinoyl (Dts) amino acids and N-(iso-propyldithio)carbonylproline, together with p-alkoxybenzyl ester anchoring linkages", & INT. J. PEPT. PROTEIN RES. 1987, 30(2), 177-205
- CHEMICAL ABSTRACTS, vol. 109, no. 3, 18th July 1988, pages 660-661, abstract no. 23352z, Columbus, Ohio, US; F. ALBERICIO et al.: "An acid-labile anchoring linkage for solid-phase synthesis of C-terminal peptide amides under mild conditions", & INT. J. PEPT. PROTEIN RES. 1987, 30(2), 206-16
- CHEMICAL ABSTRACTS, vol. 105, no. 1, 7th July 1986, page 638, abstract no. 6797y, Columbus, Ohio, US; D. BELLOF et al.: "A new phenacyl-type handle for polymer supported peptide synthesis", & CHIMIA 1985, 39(10), 317-20
- CHEMICAL ABSTRACTS, vol. 99, no. 3, 18th July 1983, page 660, abstract no. 22889t, Columbus, Ohio, US; R. COLOMBO et al.: "4-Chloromethylphenoxyacetyl polystyrene and polyamide supports for solid-phase peptide synthesis", & INT. J. PEPT. PROTEIN RES. 1983, 21(2), 118-26
- CHEMICAL ABSTRACTS, vol. 98, no. 15, 11th April 1983, page 689, abstract no. 126602q, Columbus, Ohio, US; R.C. SHEPPARD et al.: "Acid-labile resin linkage agents for use in solid phase peptide synthesis", & INT. J. PEPT. PROTEIN RES. 1982, 20(5), 451-4
- JOURNAL OF THE CHEMICAL SOCIETY, CHEMICAL COMMUNICATIONS, no. 13, 5th July 1987, pages 539-540; E. ATHERTON et al.: "Application of polyamide resins to polypeptide synthesis: an improved synthesis of beta-endorphin using fluorenylmethoxycarbonylamino-acids"
- JOURNAL OF THE CHEMICAL SOCIETY, PERKIN TRANSACTIONS I, vol. 4, no. 2, 1981, pages 538-546; E. ATHERTON et al.: "Peptide synthesis. Part 2. Procedures for solid-phase synthesis using Nalpha-fluorenylmethoxycarbonylamino-acids on polyamide supports. Synthesis of substance P and of acyl carrier protein 65-74 decapeptide"
- TETRAHEDRON LETTERS, vol. 28, no. 46, 1987, pages 5647-5650, Pergamon Press, Oxford, GB; G. BREIPOHL et al.: "Solid phase synthesis of peptide aminoalkylamides development and application of new linking agent"
- TETRAHEDRON LETTERS, vol. 30, no. 33, 1989, pages 4341-4344, Pergamon Press, Oxford, GB; M.S. BERNATOWICZ et al.: "An efficient method for racemization free attachment of 9-fluorenylmethyloxycarbonyl-amino acids to peptide synthesis supports"

## Description

### Background of the Invention

A peptide is a natural or synthetic substance characterized by a specific sequence of component amino acids which are chemically linked together by amide bonds. A widely used approach to the chemical synthesis of peptides is known as the solid-phase method. (See Barany et al., Int. J. Peptide Protein Res. 30:705-39 [1987].) The feasibility of solid-phase synthesis was first demonstrated using an amino acid protected with carbobenzyloxy (Cbz) which was immobilized on a polystyrene compound via a benzyl ester linkage. (Merrifield, R.B., J. Am. Chem. Soc. 85:2149-54 [1963].) Since then, Merrifield has also used t-butyloxy-carbonyl (Boc) for Nα protection.

A critical step in the synthesis of peptides by the solid-phase method is the covalent attachment, or "anchoring", of the first (C-terminal) amino acid to the solid support in a specific and controlled manner. When the urethane protecting group-based synthesis schemes were employed for solid-phase peptide synthesis, there were specific problems with the carboxyl group activation methods for anchoring the C-terminal amino acid (Atherton, E. et. al., J. C. S. Chem. Commun. 336-7, [1982]; Sieber, P. Tet. Lett. 28, 6147-50 [1987]; Albericio, F. and Barany, G. In. J. Peptide Protein Res. 26, 92-7 [1985]). These methods specifically suffer from low functionalization and/or partial racemization. Although solutions to such problems have been proposed (Sieber, P. Tet. Lett. 28:6147-50 [1987]; Albericio, F. and Barany, G., Int. J. Peptide Protein Res. 26:92-7 [1985]; Kirstgen, R. et al., J. Chem. Soc., Chem. Commun., 1870-1 [1987]), none of these are entirely satisfactory for industrial scale production of amino acids anchored to solid supports.

Drawbacks of these solid phase methods using carboxyl group activation include the inavailability or expense of, and/or instability of the reagents required for carboxyl group activation. In addition, esterification via carboxyl group activation in some of these cases appears to require long reaction times for optimum yields. Finally, the ability to generalize these methods to all of the amino acids of common interest has not yet been demonstrated.

9-fluorenyl methyloxy carbonyl (Fmoc) protected amino acids have been used to prepare solid-phase bound amino acids. Racemization of the amino acid from one to about twenty percent has been observed by the currently used method for attachment of Nα-Fmoc amino acids to solid supports (Atherton E. et. al., J. C. S. Chem. Commun. 336-337 [1981]). In another approach, amino acids are esterified directly to alkyl halides or polymeric alkyl halides without racemization. These methods require the laborious and expensive preparation of protected amino acid cesium salts. For Fmoc-amino acid cesium salts, the method (Columbo) has not been generally demonstrated, presumably due to poor solubility of some of these derivatives (e.g., Mitchell, A.R. et al., J. Org. Chem. 43: 2845-52 [1978]; Wang, S.S. et al., J. Org. Chem. 42:1286-90 [1977]; Columbo, R., et al., Int. J. Peptide Protein Res. 21:118-26 [1983]). Alternate methods for production of solid support derivatives which utilize benzyl ester formation via reaction of activated carboxyl groups with benzyl alcohol derivatives in the presence of acylation catalyst have been shown to produce measurable amounts of racemization of the protected amino acid (Dhaon, M.K. et al., J. Org. Chem. 47:1962-1965 [1981]; Atherton, E. et al., J.C.S. Chem. Commun. 336-337 [1981]).

In CHEMICAL ABSTRACTS, vol. 109, no. 5, August 1, 1988, page 658, abstract no. 38219k there is disclosed that several Nα-dithiosuccinoyl 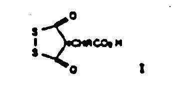 (Dts) amino acids I [R = H, Me, CHMe₂, CH₂CHMe₂, CH₂Ph, CH₂CH₂SMe, (CH₂)₃NHC(NHNO₂):NH] have been esterified without racemization by use of either DCC or Me₂N(CH₃)₃N:C:NEt,HCl, each in the presence of 4-dimethylaminopyridine to 4-HOCH₂C₆H₄O-(CH₂)ₙCO₂C₄H₂Cl₃-2,4,5 (n = 2,3). The resultant handle derivatives, were purified and then quantitatively attached onto aminomethyl supports by couplings in DMF containing 1-hydroxybenzotriazole. This methodology facilitates anchoring of Dts-amino acids as p-alkoxybenzyl esters, which can be cleaved in good yields by 1:1 CF₃CO₂HCH₂Cl₂ at 25°.

CHEMICAL ABSTRACTS, vol. 109, no. 3, July 18, 1988, page 660 to 661, abstract no. 23352z discloses that a series of polymer-supported benzylamides 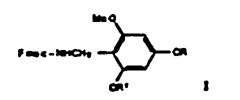 substituted with 1 to 3 alkoxy groups in the ring positions were prepared and shown to give carboxamides upon treatment with acid. Based on the initial screening, the bis(o-methoxy)-p-alkoxybenzylamide anchoring linkage was selected for a detailed evaluation of its suitability for solid-phase synthesis of C-terminal peptide amides.

In CHEMICAL ABSTRACTS, vol. 105, no. 1, July 7, 1986, page 638, abstract no. 6797y there is disclosed that phenoxyacetate I (R = OH) (II) was prepared as a new 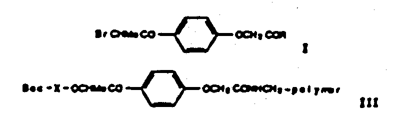 phenacyl-type handle for polymer-supported peptide synthesis. II was condensed with H₂NCH₂-polymer by DCC to give I (R = NHCH₂-polymer), which were esterified with Boc-X-OH [Boc = Me₃CO₂C; X = Gly, Leu, Tyr(CH₂Ph), Leu] via KF/DMF or its Cs salt to give polymer-bound amino acids III. III can be used for solid-phase peptide synthesis; the final peptide can be readily cleaved from the phenacyl polymer by nucleophiles or photolysis.

In CHEMICAL ABSTRACTS, vol. 99, no. 3, July 18, 1983, page 660, abstract no. 22889t there is disclosed that 4-chloromethylphenoxyacetamidomethylcopoly (styrene-1%-divinylbenzene) and 4-chloromethylphenoxyacetyl-norleucylpoly(dimethylacrylamide) were prepaared as functionalized supports for the solid-phase synthesis of peptides under mild conditions in order to avoid the danger of racemization during base-catalyzed esterification of the first protected amino acid to the 4-alkoxybenzyl alc. resins formerly employed in combination with Nα-9-fluorenylmethoxycarbonyl and tert-Bu side-chain protecting groups. Esterification of Nα-protected amino acids to the new resins can be achieved easily and without significant levels of racemization by means of their cesium salts, while cleavage from the supports is possible by treatment with CF₃CO₂H.

In CHEMICAL ABSTRACTS, vol. 98, no. 15, April 11, 1983, page 689, abstract no. 126602q there is disclosed that acid-labile 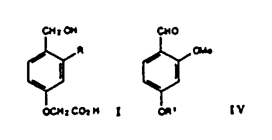 peptide-resin linkage agents I (R = H, OMe) were prepared from 4-formylphenol (II) and 3-methoxyphenol (III), respectively. Thus, III was formylated with POCl₃/DMF to give benzaldehyde IV (R¹ = H), which was treated with ClCH₂CO₂H in the presence of aq. NaOH to give IV (R¹ = CH₂CO₂H), which was reduced by NaBH₄ to give I (R = OMe). II was treated with ClCH₂CO₂H to give 4-HCOC₆H₄OCH₂CO₂H, which was reduced by NaBH₄ to give I (R = H). I (R = OMe) is suitable for the preparation of peptide fragments having tert-Bu-based side-chain protective groups.

FR-A-2 286 811 discloses p- or o-chloromethylbenzoic acids of phenol or thiophenol represented by the following formula (I): 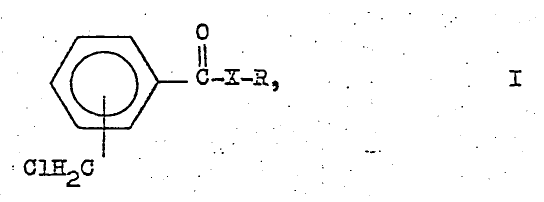 wherein X represents an oxygen or sulfur atom and R represents an unsubstituted or substituted, monocyclic or polycyclic phenol residue the substituent(s) comprising the following substituent or a corresponding thiophenol thereof.

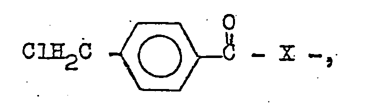

E. Atherton et al. disclose in Journal of the Chemical Society; Chemical Communications, no. 13, July 5, 1987, pages 539 to 540 a new procedure which employs base labile N-α-fluorenylmethoxycarbonyl (Fmoc) amino-acids in combination with acid-labile t-butyl based side chain protecting groups and p-alkoxybenzyl ester resin linkage in order to produce under milder reaction conditions by use of a solid phase peptide synthesis procedure human β-endorphin.

E. Atherton et al. disclose in Journal of the Chemical Society; Perkin Transactions I, vol. 4, no. 2, 1981, pages 538 to 546 the use of base-labile Nα-9-fluorenylmethoxycarbonylamino-acids in combination with acid-labile t-butyl or p-alkoxybenzyl side-chain or carboxy-terminal (resin-linkage) protecting groups enabling solid-phase peptide synthesis to be carried out under exceptionally mild reaction conditions. Using this new combination of protecting groups on polyamide supports high-yield syntheses of a decapeptide and of an undecapeptide amide (Substance P) are possible to be carried out.

There exists a need for a general and inexpensive means for controlled and efficient anchoring of C-terminal amino acids to solid supports without significant racemization of the amino acids.

### Summary of the Invention

This invention pertains to a method according to claims 13 or 16 for covalently linking amino acids to solid supports in a racemization free manner for use in solid phase peptide syntheses. This invention also pertains to new compounds according to claim 1 which are used to covalently and specifically link amino acids to solid supports. This invention further pertains to a method according to claim 19 for synthesizing peptides from the solid-support bound amino acid derivatives produced by the methods of this invention.

According to the method of this invention, a protected amino acid covalently bound to a solid support can be prepared having the general formula (I): 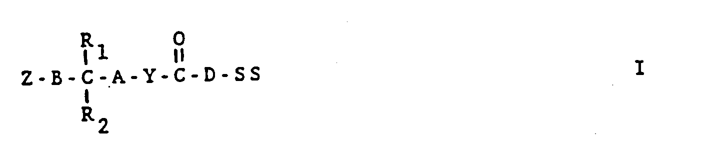
wherein Z is an Nα amino acid protecting group other than dithiosuccinyl or (isopropyldithio)carbonyl;
B is a naturally occurring D or L amino acid having protected or unprotected side chains;
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage;
D is an oxygen atom or -NH- which may be linked to a spacer selected from the group consisting of a natural or unnatural amino acid and a hydrocarbon chain having a linear or branched, saturated or unsaturated alkyl, aryl or aralkyl group, the spacer being linked to the carbonyl through an oxygen or -NH- linkage; and
SS is a solid support which is bound to the carbonyl group through D and is selected from the group consisting of polystyrene crosslinked with divinylbenzene, crosslinked polydimethylacrylamide, glass, controlled pore glass, polyvinylidinedifluoride, polypropylene, polyethylene, ceramics, silicas and aluminas.

### Brief Description of the Drawings

Figure 1 is a schematic representation for racemization free attachment of C-terminal amino acids to solid supports for peptide synthesis.
Figure 2 is an alternate schematic representation for racemization free attachment of C-terminal amino acids to solid supports for peptide synthesis.
Figure 3a shows an HPLC chromatogram of GITC derivatives of a standard D,L mixture of phenylalanine.
Figure 3b shows an HPLC chromatogram of GITC derivatives obtained from a resin prepared by the methods of Atherton (J.C.S. Chem. Comm., 336-337 [1981]).
Figure 3c shows an HPLC chromatogram of GITC derivative obtained from a resin prepared by the methods of this invention.
Figure 4a shows the HPLC chromatogram of Figure 3b as expanded by computer.
Figure 4b shows the HPLC chromatogram of Figure 3c as expanded by computer.

### Detailed Description of the Invention

This invention pertains to a method of preparing a protected amino acid covalently linked to a solid support having the general formula (I): 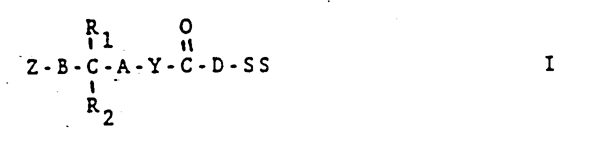 Z is an Nα amino acid protecting group other than dithiosuccinyl or (isopropyldithio)carbonyl. B is one or more naturally occuring D or L amino acids having protected or unprotected side chains. Preferably, B is one amino acid. R₁ and R₂ are the same or different and are selected from the group consisting of hydrogen, linear or branched, saturated or unsaturated alkyl, aryl and benzyl. Preferably, R₁ and R₂ are both hydrogen. A is a substituted or unsubstituted phenyl ring, wherein the substituents are selected from the group consisting of aryl, alkyl, halo, nitro, alkoxy and combinations thereof. Preferably the phenyl ring is a substituted or unsubstituted benzene ring wherein the benzene group is linked to C and Y at its para positions. Y is a hydrocarbon chain having a linear or branched, saturated or unsaturated alkyl, aryl or aralkyl group which may be linked to A through an ether linkage. Preferably Y is -(O)ₘ-(CH₂)ₙ where m is one or zero and n is an integer from above zero to twelve. D is an oxygen atom or -NH- which may be linked to a spacer selected from the group consisting of one or more natural or unnatural amino acids or hydrocarbon chain having linear or branched, saturated or unsaturated alkyl, aryl or aralkyl groups, the spacer being linked to the carbonyl through an oxygen or -NH- linkage. SS is a solid support which is bound to the carbonyl group through D and is selected from the group consisting of polystyrene cross-linked with divinylbenzene, crosslinked polydimethylacrylamide, glass, controlled pore glass, polyvinylidinedifluoride, polypropylene, polyethylene, ceramics, silicas and aluminas. Preferable solid supports are Pepsyn™, Pepsyn K™ and polystyrene resins.

The method of the present invention provides an economical means for producing derivatized solid supports which can be used in solid phase peptide synthesis. The method makes use of starting materials which are inexpensive and react at rapid reaction rates. Further, the present invention eliminates the need to use cesium salts of the Nα protected amino acids such as Boc-amino acid-O-Cs⁺ as used in the Mitchell method, or expensive and toxic additives such as 18-crown-6 as used in the Columbo method.

The method of the present invention provides a means for practical, controlled and efficient anchoring of C-terminal amino acids to solid supports without incurring racemization of the amino acids. Thus, peptides produced using the solid phase-bound amino acids of the present invention are produced in better yield and purity than peptides produced using conventional solid phase supports.

The above compound can be synthesized by two schemes detailed below. The intermediates of these syntheses are also novel compounds. A more detailed description of the reactants suitable for these syntheses follows below.

### Scheme I

In a first scheme, carboxylic acid (II) with leaving group X (other than Cl) undergoes a condensation reaction with an alcohol, phenol, thiol, thiophenol or heterocyclic equivalent in the presence of a condensing agent to yield the compound (III). 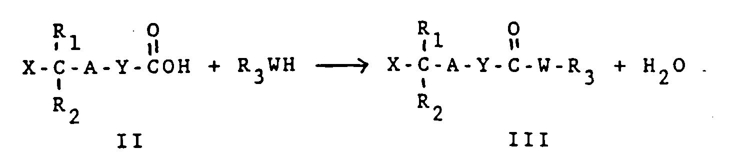 A, R₁, R₂ and Y are as defined above. X is a leaving group other than Cl which can be readily displaced by a carboxylate in a subsequent synthetic step. A leaving group as defined herein is any electron withdrawing substituent which renders the carbon atom to which it is attached electrophilic thereby facilitating nucleophilic substitution reactions at that carbon. Preferable leaving groups are any halogen other than Cl but may also be a sulfonyl group such as methane sulfonyl, trifluoromethyl sulfonyl or p-toluene sulfonyl. In a preferred embodiment, compound (II) is 4-bromomethylphenoxyacetic acid or 4-iodomethylphenoxyacetic acid. W is either a sulfur atom or an oxygen atom. R₃ represents a group derived from the alcohol, phenol, thiol, thiophenol, or analogous heterocyclic alcohol or thiol. Any alcohol, phenol, thiol, thiophenol, or analogous heterocyclic alcohol or thiol can be used as a reagent so long as R₃ acts as a carboxyl protecting group. R₃ may or may not activate the carboxyl group to nucleophilic attack. If R₃ is such an activating group, it must produce compound (III) of sufficient stability such that R₃ is not transferred to the carboxyl group of the amino acid used in the subsequent synthetic step, yet it must be sufficiently reactive to render compound (IV) susceptible to nucleophilic displacement by atom D attached to a solid support to yield the compound of Formula I. Where R₃ is not a carbonyl activating group, it must be capable of removal under conditions which do not affect the Nα and side chain protecting groups.

An Nα protected amino acid is then esterified directly to the substituted aromatic ester (III) to yield the compound of the following formula: 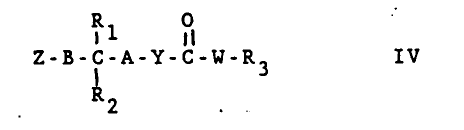 wherein R₁, R₂, Y, A, W and R₃ have been previously defined. B represents an amino acid or peptide linked through its carboxyl group to the benzylic carbon of the compound. The amino acid can be a naturally occuring amino acid or modified versions thereof. Although B is shown as a single amino acid, it should be understood that B can represent more than one amino acid. The amino acid side chain may be optionally or necessarily protected with a side chain protecting group. Z is an Nα amino acid protecting group other than dithiosuccinyl or (isopropyldithio)carbonyl. The term Nα amino acid protecting group is intended to mean a structure that can bind to the amino acid through the α-nitrogen atom and thus render the amino group unreactive. The Nα amino acid protecting group should also be capable of being removed without destroying any peptide linkage produced by the methods of this invention. Preferrably, Z is a base labile protecting group such as 9-fluorenylmethyloxycarbonyl protecting group (Fmoc).

In the esterification step, Nα protected amino acid carboxylate anions are generated in solution and, as such, act as a nucleophile. An ester bond is then formed with the benzylic carbon of the substituted aromatic ester (III) by nucleophilic attack of amino acid carboxylates.

In the final step of the synthesis, the Nα protected amino acid ester (IV) is coupled directly to a solid support using a catalyst to yield the compound of formula (I) as previously described.

In a preferred embodiment, a halogenated or sulfonated carboxylic acid (V) undergoes a condensation reaction with 2,4-dichlorophenol in the presence of a condensing agent to yield the compound (VI): 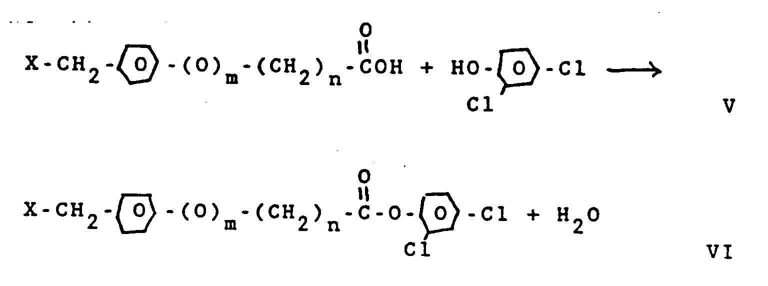 m is zero or one and n is an integer from above zero to twelve. The compound of (VI) is then esterified to an Nα protected amino acid to yield the compound of the following formula: 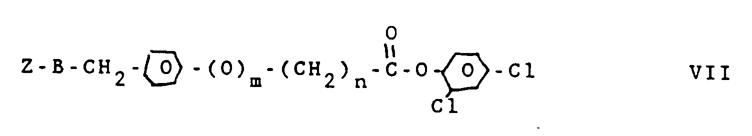 wherein Z, B, m and n have been previously defined. The compound (VII) is then coupled directly to a solid support to yield the compound of the formula: 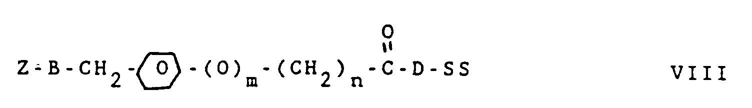 wherein the variables have been defined previously.

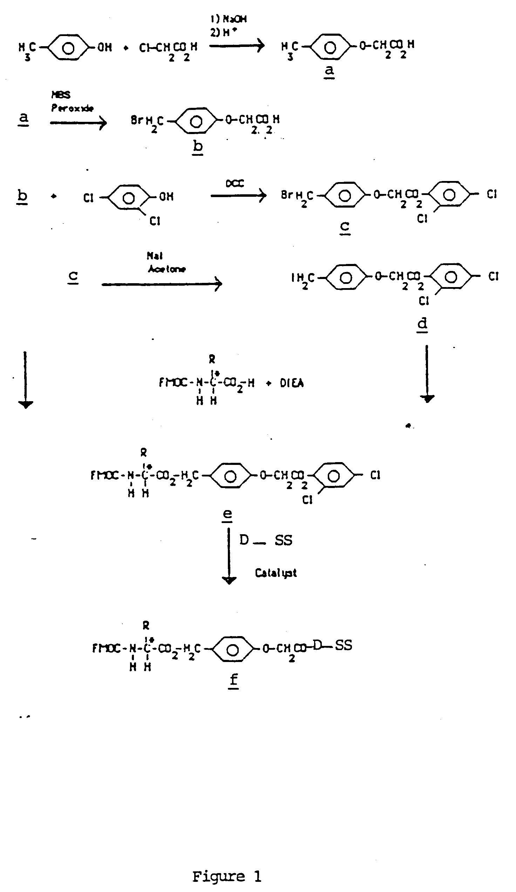

The substituted aromatic carboxylic acid, 4-methylphenoxyacetic acid is used as a starting reactant; however, other substituted aromatic carboxylic acids may be used. The abbreviations used in Figure 1 are explained as they are referred to in the following text which describes the process.

In the first step, 4-methylphenoxyacetic acid (a) is prepared by alkylation of p-cresol with chloroacetic acid in the presence of a suitable base, such as sodium hydroxide. Other isomers of 4-methylphenoxyacetic acid may be obtained using o-cresol or m-cresol. Alternate alkylating agents such as iodoacetic acid, bromoacetic acid or the equivalent thereof may be used. The reaction may be carried out in aqueous or suitable organic solvent or mixtures thereof and other bases such as potassium or lithium hydroxide, potassium carbonate, cesium carbonate, sodium hydroxide, sodium hydride, potassium hydride, or the equivalent thereof may be used to form the cresolate anion required for reaction. Acidification of the reaction mixture for product isolation is accomplished with aqueous hydrochloric acid, sulfuric acid, potassium hydrogensulfate or an equivalent proton source thereof.

In the subsequent synthetic step, 4-methylphenoxyacetic acid (a) is brominated by N-bromosuccinimide (NBS) to form 4-bromomethyl-phenoxyacetic acid (b) using chloroform or carbon tetrachloride as a solvent. The reaction is carried out in refluxing solvent in the present of a free-radical initiator such as benzoyl peroxide or an equivalent thereof. Alternatively, the reaction may be carried out using other brominating agents such as N-bromoacetamide or equivalents thereof and may be initiated by light. Similarly, alkanes, bromoform, carbon tetrabromide or an equivalent thereof may be used as solvent.

A suitable active ester of 4-bromomethylphenoxyacetic acid (b) is formed in the following synthetic step. This step is accomplished by condensation of b with 2,4-dichlorophenol using dicyclohexylcarbodiimide (DCC), as condensing agent. The reaction may be carried out in a variety of organic solvents such as ethyl acetate, methylene chloride, chloroform, dioxane, tetrahydrofuran, dimethylformide or an equivalent thereof. Other substituted phenols such as pentafluoro, pentachloro, 2-nitro, 2,4-dinitro, 4-nitro, 2-chloro, 4-chloro, 2,4,5-trichloro, 2-bromo, 4-bromo, isomers of these or their equivalents may be used to provide active ester derivatives analogous to 2,4-dichlorophenyl-4-bromomethyl phenoxy acetate (c). Other analogous esters may be obtained by substituting appropriately structured alcohols, such as trichloroethanol, phenacyl alcohol, N-hydroxy compounds such as N-hydroxysuccinimide, hydroxyl substituted heterocycles, or their equivalents, for 2,4-dichlorophenol in this reaction. In addition, thioester analogs of (c) may be obtained by condensation of b with appropriately substituted thiols. For the conversion of b to c, or b to the above-stated analogs of c, alternative condensing agents or condensation chemistries may be employed. For example, other carbodiimides such as diisopropylcarbodiimide or its equivalent may be used. Similarly, carboxyl activation of b may be accomplished by conversion of b to its acid chloride via thionyl chloride or its equivalent, or conversion to a mixed anhydride via isobutylchloroformate, pivaloyl chloride, or their equivalents. Other known methods of carboxyl group activation may be employed.

In some instances the conversion of c to its iodide derivative, 2,4-dichlorophenyl-4-iodomethylphenoxy acetate (d) may be advantageous before performing the subsequent chemical reactions depicted in Figure 1. The halogen exchange reaction is carried out by addition of saturated anhydrous sodium iodide in acetone to the bromomethyl-active ester (c). For this reaction, potassium iodide, or an equivalent iodide may be substituted for sodium iodide and tetrahydrofuran or an equivalent may be substituted for acetone as solvent.

In the next step of the synthetic scheme, either the bromide (c) or iodide (d) derivative is used to alkylate Fmoc-amino acid carboxylate anions which are generated in solution by neutralization with a tertiary amine base such as diisopropylethylamine (DIEA). Fmoc refers to the 9-fluorenyl methyloxycarbonyl protecting group and R refers to the structure of the amino acid side chain and is dependent on the particular amino acid selected. For example, when R=CH₃, the amino acid is alanine (Ala), when R=CH(CH₃)₂, the amino acid is valine (Val), etc. The R, amino acid side chain, may include the structure of a protecting group, where necessary. The asterisk at the α-carbon is used to designate this atom as a chiral center. Thus, D or L enantiomers of each amino acid may be used for this reaction. The stereochemistry is completely retained during the conversion of halides c or d to the product, Nα-9-fluorenylmethyloxycarbonylaminoacyl-4-oxymethylphenoxyacetic acid-2,4-dichlorophenylester (e). Thus, when optically pure, Fmoc-protected L-amino acids are used in the esterification, the products (e) obtained are of the L configuration and are uncontaminated with D isomer.

The esterification may be carried out in a variety of solvent or solvent mixtures including dimethylacetamide, dimethylformamide, N-methylpyrolidone, acetone and equivalents thereof. Alternate bases or hydrogen-bonding chemicals may be used to generate an effective protected amino acid carboxylate. These include triethylamine, tributylamine, cesium or potassium carbonate or bicarbonate, N-methylmorpholine, potassium fluoride, or equivalents thereof. Preferrably, Fmoc is used as the Nα-protecting group; however, other protecting groups may be used for peptide synthesis such as t-butyloxycarbonyl (Boc), carbobenzyloxycarbonyl (Cbz), 2-nitropyridinesulfenyl (Npys), trifluoroacetyl, 2(p-biphenylisopropyloxycarbonyl (Bpoc), 4-methoxybenzyloxycarbonyl (Pmz), tosyl, trityl, methoxy-substituted trityls, 9-phenylfluorenyl, 2-nitrophenylsulfenyl (Nps), benzyl, p-nitrocarbobenzyloxycarbonyl (p-NO₂Cbz), t-amyloxycarbonyl (Aoc), 2-(3,5-Dimethyloxyphenyl)-propyl-(2)-oxycarbonyl (Ddz), 2,2-[bis(4-nitrophenyl)]-ethyloxycarbonyl (Bnpeoc), adamantyloxycarbonyl (Adoc). 2,2,2-trichloroethyloxycarbonyl (Troc), and variously substituted analogs and isomers and their equivalents thereof.

Table 1 lists various carboxy-functionalized Nα-Fmoc protected amino acids (e) that have been prepared via iodide (d) or bromide (c) derivatives and the appropriate Nα-Fmoc-amino acids as indicated in Figure 1. Proton NMR spectra of all the derivatives listed in Table 1 were consistent with the expected structures. The process is compatible with a complete range of side chain protecting groups useful in peptide synthesis. In addition to the side chain protecting groups indicated in Table 1 (for abbreviations, see Table 1 footnote), the process may be used with other side chain protecting groups, such as Cbz, various chloro, bromo, and methyl substituted Cbz groups, Pmz, p-NO₂Cbz, Aoc, Adoc, Bpoc, trifluoracetyl, Nps, Npys, tosyl, variously substituted tosyls, Ddz, Bnpeoc, benzyl and various chloro, bromo, nitro, and methoxy substituted benzyls, dinitrophenyl (Dnp), t-butyloxymethyl (Bum), benzyloxymethyl (Bom), substituted trityls, 9-phenyfluorenyl, substituted t-butyls, trichloroethyl, nitro benzyl, Troc, phenacyl, 2,2,5,7,8-pentamethylchroman-6-sulfonyl (Pmc), benzydrylamine (BHA), methylbenzydrylamine (Mbha), xanthyl (Xan), 2,4,6-trimethoxybenzyl (Tmob) and other variously substituted analogs, isomers or equivalent groups thereof.

**TABLE 1**

| Fmoc-Amino Acid Derivative (e) | | % Yield | m.p. (°C) |
|---|---|---|---|
| 1. | Ala | 99^{a} | 120-2 |
| 2. | Leu | 76 | 75-81 |
| 3. | Val | 89 | 74-7 |
| 4. | Pro | 74^{a} | 62-5 |
| 5. | Trp | 80^{a} | 136-9 |
| 6. | Met | 75 | 92-5 |
| 7. | Lys (Boc)^{c} | 90 | 75-8 |
| 8. | Phe | 79 | 101-3 |
| 9. | Glu (tBu) | 84 | 61-5 |
| 10. | Ser (tBu) | 78^{a} | 90-95 |
| 11. | Thr (tBu) | 80^{a} | amorphous solid^{b} |
| 12. | Cys (Trt) | 78^{a} | amorphous solid^{b} |
| 13. | Arg (Mtr) | 86^{a} | amorphous solid |
| 14. | Ile | 87 | 120-5 |
| 15. | Asn (Mbh) | 61 | 193-5 |
| 16. | Gln (Mbh) | 91 | 159-65 |
| 17. | His (Boc) | 76 | amorphous solid^{b} |
| 18. | Asp (tBu) | 85 | 85-9 |
| 19. | Gly | 83 | 80-90 |
| 20. | Tyr (tBu) | 63 | 109-11 |
| 21. | Cys (tBu) | 93 | 95-8 |
| 22. | Cys (Acm) | 82 | amorphous solid |
| 23. | Asn | 75 | amorphous solid |
| 24. | Gln | 74 | amorphous solid |

| | | | |
|---|---|---|---|
| a. Crude yield | | | |
| b. These materials produce "foams" under high vacuum which can be physically broken down to give easily handled, apparently stable powders. | | | |
| c. Abbreviations used for side-chain protecting groups (in parentheses) are: Boc, t-butyloxycarbonyl; tBu, t-butyl; Trt, trityl; Mtr, 4-methoxy-2,3,6-trimethylbenzenesulfonyl; Mbh, 4,4′-dimethoxybenzhydryl; Acm, acetamidomethyl. | | | |

In the final step (Figure 1), functionalized Nα-Fmoc-amino acid esters (e) are coupled directly to solid supports, designated by D-SS, where D is some nucleophilic group, and may be either amino or hydroxyl to yield the compound (f). The coupling to the support is facilitated by the use of a catalyst. The catalyst may be pyridine, 4-dimethylaminopyridine (DMAP), N-methylimidazole, 1-hydroxybenzotriazole (HOBT), or their equivalent. The coupling reaction may be carried out using dimethyformamide (DMF), dimethylacetamide (DMA), N-methylpyrolidone (NMP), methylene chloride, or mixtures of these or their equivalent. The solid supports functionalized by this process may be those based upon materials of current widespread use in peptide synthesis including 1% divinylbenzene (1% DVB) - crosslinked polystyrene, crosslinked polydimethylacrylamide (commercially available Pepsyn and Pepsyn K resins), equivalents thereof containing various degrees of crosslinking and functionalization, functionalized controlled-pore glasses, as well as experimental polymers such as functionalized polyvinylidinedifluoride or polypropylene based membranes, functionalized silicas or aluminas, and ceramic materials, or other insoluble materials onto which a nucleophilic group may be incorporated.

### Scheme II

The solid support bound amino acid of formula I can be synthesized using an alternate scheme. In this scheme, a linker is attached to the solid support prior to esterification of the amino acid. The term linker as defined herein is a portion of the compound between the amino acid and the solid support which links the two together. During esterification, the benzylic leaving group undergoes nucleophilic attack by the carboxylate of the amino acid. This esterification also results in the racemization-free attachment of amino acids to solid supports.

In the second scheme, compound II is treated with a hydroxide source to convert X to a hydroxyl group. The resulting compound is then condensed with a solid support, optionally in the presence of a catalyst, to form the solid support linked compound of the formula (IX).

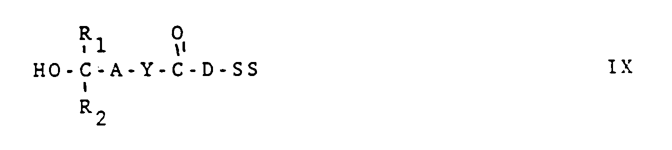

The hydroxyl group of compound IX is then converted to a leaving group such as a halogen other than Cl or a sulfonyl. The resulting compound undergoes displacement of the leaving group with an Nα protected amino acid to yield the compound of formula I or the preferred compound (VIII) as previously described.

Preferably, a halogenated aromatic carboxylic acid analog is reacted with a hydroxide source yielding a hydroxy substituted aromatic carboxylic acid (X). 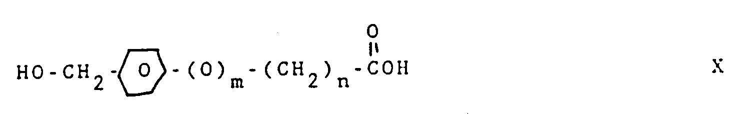 Compound (X) is then condensed to a functionalized support to yield the compound of formula (XI): 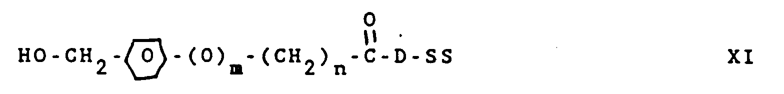 The hydroxyl group on compound (XI) is then replaced by a leaving group X other than Cl. In a subsequent step, the leaving group is displaced by an Nα protected amino acid to yield the compound of formula VIII.

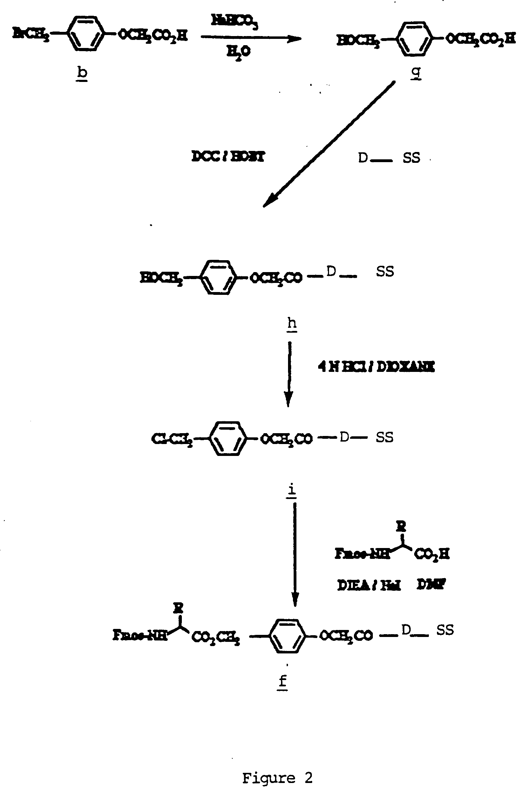

In Scheme II, bromo-acid (b) was used as the starting material to facilitate the racemization-free attachment of amino acids to peptide synthesis solid supports as seen in the schematic of Figure 2.

In the initial step, bromo-acid (b) is converted to hydroxy-acid (g) by the action of aqueous NaHCO₃. Other aqueous bases (hydroxide source) buffered to the appropriate pH may be used for this conversion. These include lithium, sodium, potassium, cesium, magnesium, calcium, and barium salts of hydroxide, carbonate, bicarbonate, or their equivalent. In the subsequent step hydroxy-acid (g) is condensed with a solid support containing some nucleophilic functionality, D, which may be hydroxyl or amino. The condensation may be performed using carbodiimides such as dicyclohexyl carbodiimide (DCC) with, or without, 1-hydroxybenzotriazole (HOBT) as a catalyst. Other acylation catalysts such as N-hydroxysuccinimide or its equivalent may be used. Active ester derivatives of (g) may also be used for condensation with the solid support to produce functionalized supports (h). Other known modes of carboxyl activation, as previously discussed in relation to the scheme of Figure 1, may be used to prepare derivatives (h). A key step in this process is the conversion of hydroxymethyl supports (h) to chloromethyl supports (i). This gas accomplished by reaction of h with 4N HCl in dioxane at room temperature. Solvents other than dioxane such as ethyl acetate, tetrahydrofuran, acetic acid, methylene chloride, ether, water, or mixtures of these solvents or their equivalent may be used for the chlorination. Other known reagents and methods of replacement of a hydroxyl group by halogen or other leaving group may be employed. Such reagents include PCl₃, PCl₅, POCl₃, triphenylphosphine in CCl₄ or CBr₄, HBr in an appropriate solvent, PBr₃, aryl and alkylsulfonyl chlorides or bromides in solution with an appropriate base such as pyridine or diisopropylethy]amine (DIEA) or their equivalances. In the final step, the protected amino acid other than a protected amino acid caesiun salt is esterified to the resin by nucleophilic displacement of the leaving group previously incorporated In this case, the chloromethyl group of (i) was allowed to react at room temperature with an Fmoc-amino acid in dimethylformamide (DMF) containing DIEA and NaI. In the case of Fmoc-alanine, the coupling yield under these conditions was >98% of theoretical and the Fmoc-alanine thus incorporated was quantitatively released by treatment with trifluoroacetic acid. Other known methods for accomplishing esterification via a nucleophilic displacement reaction of a leaving group by a carboxylate, as in the conversion of (i) to (f), may be employed. These have been previously discussed in the description of the chemistry used in Scheme 1. Similarly, other protected amino acids, as previously described in relation to Scheme 1 can be used as starting materials for the esterification.

### Racemization Analysis

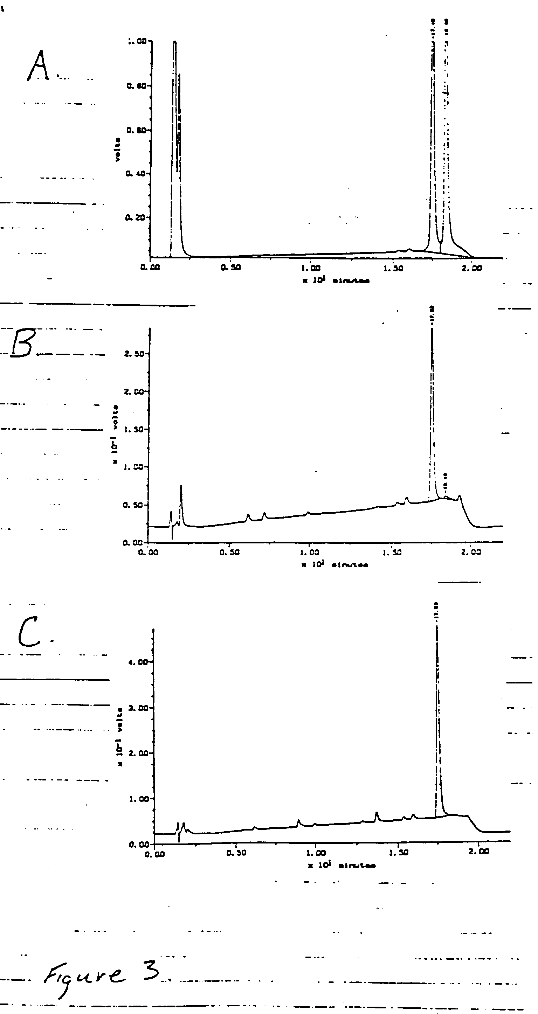

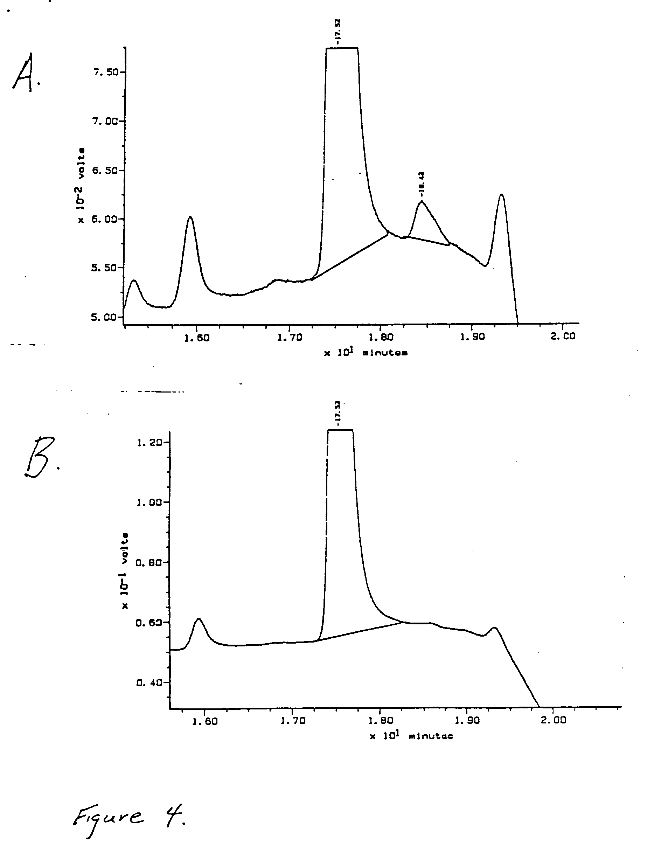

In order to demonstrate the optical purity of the solid support bound amino acids prepared by the processes described herein, a chiral derivatizing agent, 2,3,4,6-tetra-O-acetyl-β-D-glucopyranosyl isothiocyanate (GITC), (Nimura, N. et al. Journal of Chromatography 202:375-9 [1980]; Kinoshita, T. et al. Journal of Chromatography 210:77-81 [1981]) which is capable of converting D,L amino acid mixture into mixtures of reverse phase HPLC resolvable diastereomers, was used. In these experiments Pepsyn-K based resins prepared by the new processes described herein were compared with commercially available resins prepared by current art methods (Atherton, E. et al., J.C.S. Chem. Commun. 336-337 [1981]) and commercially available D,L-amino acid standard mixtures (Sigma Chemical Co., St. Louis, MO). After removal of the Fmoc group from the Fmoc-amino acid-Pepsyn K resins with 20% piperidine in DMF (v/v), followed by washout of excess reagent with DMF then CH₂Cl₂, the free amino acids were cleaved from the resins with neat trifluoroacetic acid (TFA). The TFA was separated from the resins and evaporated to dryness to give the free amino acids as dry TFA salts. The free amino acids were derivatized with GITC reagent as described in the literature (Nimura, N, et al., Journal of Chromatography 202:375-9 [1980]; Kinoshita, T. et al., Journal of Chromatography 210:77-81 [1981]). The results of the HPLC analysis (C-18 column, 15-minute linear gradient of 20 to 40% acetonitrile in water containing 0.1% TFA) of the amino acids obtained in this manner from Fmoc-Phe-Pepsyn K resins, prepared by the methods of this invention and commercial Fmoc-Phe-Pepsyn K product (Atherton, E. et al., J.C.S. Chem. Commun. 336-337 [1981)) are shown in Figures 3 and 4. The phenylalanine derivative obtained from the resin prepared by the methods of this invention was essentially free of D-isomer contaminant and is therefore consistent with lack of racemization. In contrast, the commercial phenylalanine derivative was found to contain a substantial amount (2%) of D-isomer contaminant as quantitated by peak integration. Figure 3b shows a small peak eluting at 18.43 minutes which corresponds to 2% contamination by D-isomer in the commercial product produced by the methods of Atherton et al.

### Peptide Synthesis

The solid support bound amino acids of Formula I are used for the synthesis of specific peptide sequences. The solid support is chemically functionalized to anchor the first peptide building block. The synthesis of the specific peptide sequence is performed either manually or by automated synthesis. Standard chemical protocols for the stepwise construction of peptides can be employed.

Protected amino acids other than protected amino acid caesium salts can sequentially be coupled to the first amino acid bound to the solid support to produce solid support bound peptides.

Examples of such methods are Gutte, B. & Merrifield, R.B. (1971) J.Biol. Chem. 246:1922-1941; Kent, S.B., et al., (1978) Anal. Chem. 50:155-159; Wong, T.W. & Merrifield, R.B. (1980) Biochemistry 19:3233-3238; Arshady, R., et al., (1981) J. Chem. Soc. Perkin Trans. I, 529-537; Atherton, E., et al., (1981) J. Chem. Soc., Perkin Trans. I, 538-546; Stewart, J.M. & Young, J.D. (1984) in Solid Phase Peptide Synthesis, 2nd edn., Pierce Chemical Co., Rockford, IL; and Atherton, E., Sheppard, R.C. & Ward, P. (1985) J. Chem. Soc., Perkin Trans. I, 2065-2073. After the assembly of the desired specific sequence, the protecting groups can be removed to generate biologically functional molecules.

Depending on the linkage to the solid support, the synthesized peptide can either be cleaved off for subsequent characterization and/or identification or it can be left on the solid support in an unprotected form.

The present invention will now be illustrated by the following examples which are not to be considered limiting in any way.

### Example 1

### Preparation of 4-Bromomethylphenoxyacetic acid (b)

To 4-methylphenoxyacetic acid (87.3 g, 0.525 mole) and N-bromosuccinimide (112.2 g, 0.63 mole) was added 900 ml of dry CHCl₃ and 100 mg benzoyl peroxide and the mixture was refluxed for 2.5 hours. The mixture was allowed to cool and was stored overnight at 4°. The crystals which had formed were collected by vacuum filtration and were washed twice with 50 ml of ice-cold CHCl₃ followed by 50 ml washes with water. After drying in vacuo over P₂O₅ to a constant weight, 52.9 g of white product (mp 122-4°C) were obtained. To obtain additional product, the organic mother liquor was washed with water (3x150 ml), and 150 ml brine. After drying over Na₂SO₄ and filtration, the solvent was removed in vacuo and the residue crystallized from 90 ml boiling CHCl₃. The crystals were collected by vacuum filtration, washed three times with 20 ml of CHCl₃/hexanes (1:1) and dried in vacuo to give 17.6 g of additional product (mp 121-2°C). Total yield, 70.5 g (55%). The product displayed an R_{f}=0.33 upon TLC (silica gel 60) developed with 95:5 CHCl₃:acetic acid. ¹H NMR (300 MHz, CDCl₃, ppm δ relative to tetramethylsilane) : 4.41 (s,2H), 4.62 (s,2H), 6.24 (broad s, 1H), 6.82 (d, J=8.7 Hz, 2H), 7.28 (d, J=8.7 Hz, 2H).

### Example 2

### Preparation of 2,4-Dichlorophenyl-4-bromomethylphenoxy acetate (c)

To 4-bromomethylphenoxyacetic acid (20 g, 81.6 mole) and 2,4-dichlorophenol (14 g, 85.7 mole) suspended in 100 ml ethyl acetate at 0°C was added solid dicyclohexylcarbodiimide (17.7 g, 85.8 mole) in portions with stirring. After two hours at 0°C, 200 ml of ethyl acetate was added to the thick suspension that resulted, and stirring was allowed to proceed for 30 minutes as the mixture was allowed to warm to room temperature. Insoluble dicyclohexylurea was removed by filtration and was washed with ethyl acetate (3x30 ml). The combined filtrate and washes were concentrated in vacuo and the residue was dissolved in 35 ml boiling ethyl acetate. Upon cooling to room temperature, crystalline product had formed. After addition of 155 ml hexanes in portions with mixing, the product was collected by vacuum filtration, washed with hexanes/ethyl acetate (9:1), and dried in vacuo to yield 26.8 g (85%) of crystalline product (mp 114-6°C). The product was homogeneous by TLC (silica gel 60) and had an R_{f}=0.50 when developed with hexanes:ethyl acetate (8:2).
¹H NMR (300 MHz, CDCl₃, ppm δ relative to tetramethylsilane): 4.51(s, 2H), 4.98(s,2H), 6.97(d, J=10.2 Hz, 2H), 7.12(d, J=10.2 Hz, 1H), 7.28-7.4(m, 3H, includes d, J=10.2 Hz, 2H), 7.48(d, J=3.4 Hz, 1H).

### Example 3

### Preparation of 2,4-Dichlorophenyl-4-iodomethyl phenoxyacetate (d)

To 2,4-dichlorophenyl-4-bromomethyl phenoxyacetate (7.21 g, 18.48 mmole) was added to a solution of anhydrous sodium iodide (2.96 g, 19.7 mmole) in 100 ml acetone. The mixture was stirred for 30 minutes at room temperature. The insoluble sodium bromide which formed was removed by vacuum filtration and washed three times with 5 ml portions of acetone. Removal of the solvent in vacuo from the combined filtrate and washes furnished 8.1 g (100%) of a slightly yellow crystalline solid (mp 121-2°C) which was used for synthesis without further purification. The product exhibited R_{f}=0.59 on TLC (silica gel 60) developed with hexanes:ethyl acetate (1:1).

### Example 4

### Preparation of Amino Acid Derivatives (e)

To 4-iodemethyl active ester (d) (1.0 g, 2.3 mmole) and the appropriate Nα-Fmoc-amino acid (2.5 mmole) dissolved or suspended in 4 to 5 ml dimethylacetamide:acetone (1:1) was added diisopropylethylamine (0.45 ml, 2.6 mmole) dropwise with stirring at room temperature. In general, a solution results within one hour and reactions are complete within two hours as judged by disappearance of d on TLC. To the reaction mixture was added 20 ml ether:ethyl acetate (1:1) and the organic phase was washed in succession with 10 ml portions of water, 5% aqueous sodium bicarbonate, 5% aqueous sodium thiosulfate, water, brine, and dried with anhydrous MgSO₄. After filtration to remove drying agent, the products were obtained by removal of solvent and drying in vacuo. When possible, the products were crystallized from an appropriate solvent or solvent pair. In the case of the Cys(Acm), Asn, and Gln derivatives, the reactions were carried out similarly starting with the 4-bromomethyl active ester c and were conducted using neat dimethylformamide as solvent. In the case of the Asn and Gln derivatives, the products precipitated from the organic phase during the usual aqueous washes. These products were collected by filtration, washed with ether followed by several portions of water and dried in vacuo over P₂O₅.

### Example 5

### Preparation of Solid Supports Derivatized with Protected Amino Acids via a Functional Linking Group (f)

Commercial Pepsyn K™ (250 umole sarcosine methyl ester/g) resin (25 g) was treated overnight at room temperature with 60 ml ethylenediamine. The ethylenediamine was removed by filtration and the resin was washed successively six times with dimethylformamide (DMF), six times with CH₂Cl₂, three times with ether and dried in vacuo. In order to determine the resulting amine substitution level, a small portion of the ethylenediamine derivatized resin was treated with a large excess of Fmoc-alanine pentafluorophenyl ester (0.3 M in DMF) in the presence of one equivalent of 1-hydroxybenzotriazole. After one hour at room temperature, the amino groups incorporated on the resin were completely coupled to Fmoc-alanine as evidenced by failure of the resin to react with ninhydrin reagent. The resin was washed successively six times each with DMF, CH₂Cl₂, ether and dried in vacuo. The Fmoc-group incorporation was quantitated spectrophotometrically after cleavage with piperidine in CH₂Cl₂. Correction for the weight gain of the incorporated Fmoc-alanine indicated that the starting ethylenediamine derivatized Pepsyn K™ had a substitution of 0.24 mmole amino groups/g. This amino-Pepsyn K derivative was used to obtain the results reported in Table 2.

In a typical solid-support derivatization experiment, 0.5 to 1.5 g of the ethylenediamine derivatized Pepsy K resin was used. The appropriate Fmoc-amino acid derivative (e) was dissolved in an amount of DMF just sufficient to completely swell and wet the resin (about 2.5 ml/g resin) and was added to the resin together with 4-dimethylaminopyridine (exact amounts of reagents are given in Table 2). After gentle mixing to remove any entrained air bubbles, the mixture was allowed to stand at room temperature for three hours. In general, a three hour reaction time was sufficient to completely, or nearly completely, couple all the resin-bound amino groups as indicated by lack of reaction of the resin products with ninhydrin reagent. After the coupling reaction the resins were washed successively as described above for the work-up of the starting ethylenediame derivatized Pepsyn K. After drying in vacuo to constant weight, samples of the resins were subjected to spectrophotometric analysis of the Fmoc-chromophore released upon treatment with piperidine in CH₂Cl₂. The results obtained are given in Table 2.

**TABLE 2**

| Solid Supports (f) Prepared by Coupling of Amino Acid Derivatives (e) to Amino-Pepsyn-KA, 0.24 mmold-NH₂/g | | | |
|---|---|---|---|
| Starting Fmoc-Amino Acid Derivative (e) (equivalents) | | Catalyst Equivalents | Final Substitution ^{b} (mmole Fmoc/g) |
| Val | (3.0) | 0.7 (DMAP)^{a} | 0.184 |
| Phe | (3.0) | 0.7 (DMAP) | 0.196 |
| Trp | (3.0) | 0.7 (DMAP) | 0.175 |
| Ala | (3.0) | 0.7 (DMAP) | 0.187 |
| Ile | (2.0) | 0.7 (DMAP) | 0.171 |
| Glu | (2.0) | 0.7 (DMAP) | 0.166 |
| Gln (Mbh)^{a} | (2.5) | 0.7 (DMAP) | 0.170 |
| Arg (Mtr) | (2.5) | 0.7 (DMAP) | 0.181 |
| His (Boc) | (2.5) | 0.7 (DMAP) | 0.160 |
| Met | (2.5) | 0.7 (DMAP) | 0.166 |
| Cys (Trt) | (3.0) | 2.6 (DMAP) | 0.150^{c} |
| Lys (Boc) | (2.5) | 2.2 (DMAP) | 0.191 |
| Ser (tBu) | (2.5) | 2.2 (DMAP) | 0.177 |
| Thr (tBu) | (2.5) | 2.2 (DMAP) | 0.171 |
| Asn (Mbh) | (2.5) | 2.2 (DMAP) | 0.190 |
| Pro | (2.5) | 0.7 (DMAP) | 0.213 |
| Asp (tBu) | (2.5) | 0.7 (DMAP) | 0.178 |
| Gly | (2.5) | 0.7 (DMAP) | 0.186 |
| Try (tBu) | (2.5) | 0.7 (DMAP) | 0.190 |
| Leu | (2.0) | 0.75 (DMAP) | 0.195 |
| Cys (Acm) | (2.5) | 0.7 (DMAP) | 0.184 |

| | | | |
|---|---|---|---|
| a. DMAP is an abbreviation for 4-dimethylaminopyridine. Side chain protecting group abbreviations are the same as given in Table 1. | | | |
| b. Coupling time is 3 hours. | | | |
| c. A substitution level of 0.19 mmole Fmoc/g was achieved when 2.5 equivalents of Fmoc-Cys (Trt) derivative was used and 3.0 equivalents of pyridine substituted for DMAP. | | | |

### Example 7

### Analysis for Raceminazation-Free Attachment

Fmoc-amino acid derivatized Pepsyn K™ resins (100mg) (MilliGen) were placed in 16 x 100 mm glass test tubes. The resins were washed with 10 ml CH₂Cl₂ and 10 ml dimethylformamide. The washes were discarded after careful decantation from the settled resins. The Fmoc-protecting group was removed by reaction of the resins with 5 ml 20% piperidine in dimethylformamide (v/v) for 10 minutes. The resins were washed with DMF (3x10 ml) and CH₂Cl₂ (6x10 ml). Amino acids were cleaved from the resins by treatment with 5 ml neat trifluoroacetic acid for 20 minutes. The trifluoroacetic acid solutions of amino acids thus cleaved were evaporated by a stream of dry nitrogen and dried further in vacuo over solid KOH. The residues thus obtained were dissolved in 0.4 ml 50% aqueous acetonitrile (v/v) containing 0.4% triethylamine. The amino acid solutions (0.05 ml) were added to 0.10 ml of GITC solution [0.2% GITC (w/v) in acetonitrile]. After a minimum of 30 minutes reaction, aliquots (2 microliters) of GITC derivatives were subjected to HPLC analysis. The GITC derivatives thus prepared were stable in this solution for up to about 12 hours at room temperature. D,L amino acid standards were derivatized with GITC solution as described above starting with a 5 mg/ml solution of standard in 50% aqueous acetonitrile (v/v) containing 0.4% triethylamine. Injection volumes were 5 microliters.

HPLC analysis was performed using a Waters 600 gradient HPLC system with a Waters Lambda-Max Model 481 detector set at 220 nm, 1 AUFS. The column was a Waters Delta Pak™ C-18-300 A, 3.9 mm x 15 cm. Binary gradient elution buffers were: (a) 0.1% trifluoroacetic acid in water; and (b) 0.07% trifluoroacetic acid in acetonitrile.

A linear gradient of 20 to 40% buffer B at a flow rate of 1 ml/minute over 15 minutes was used. This was followed by an additional minute at 40% buffer B. The column was then allowed to reequilibrate by pumping 20% buffer B for at least 6 minutes before injection of the next sample.

### Example 8

### Preparation of 4-hydroxymethylphenoxyacetic acid (g) from 4-bromomethylphenoxyacetic acid (b)

To 4-bromomethylphenoxyacetic acid (2.50 g, 10.2 mmole) was slowly added approximately 70 ml 5% aqueous NaHCO₃ with effervescence. The resulting cloudy solution was stirred for two hours at room temperature. After filtration the solution was cooled in an ice bath and carefully acidified to pH 2 with 6 M HCl. The product was extracted with ethyl acetate (3x50 ml). The combined organic phase was washed with brine, dried over MgSO₄, filtered, and the solvent was removed by rotary evaporation. After drying in vacuo, 1.53 g (82.5%) of crude white product was obtained. After recrystallization from a mixture of ethyl acetate (10 ml) and hexanes (10 ml), 1.23 g (66%) of purified white crystalline product was obtained. mp. 112-115°C (114-116°C reported by Sheppard, R.C. and Williams, B.J., Int. J. Peptide Protein Res. 20:451-454 20:451-454 [1982])

### Example 9

### Preparation of 4-chloromethyl-Pepsyn KA™ resin derivative (i) and conversion to peptide synthesis support (f)

To 200 mg Pepsyn KA™ resin (MilliGen) (0.2 mmole hydroxymethyl groups/g) was added 1 ml of 4N HCl in dioxane. After two hours at room temperature the resin was washed thoroughly by decantation of added dimethylformamide (95x4 ml). To the resulting slurry of dimethylformamide wetted resin were added Fmoc-alanine (100 mg, 0.32 mmole), diisopropylethylamine (0.061 ml, 0.35 mmole), anhydrous sodium iodide (40 mg, 0.27 mmole), and the mixture was gently mixed by periodic manual shaking. The mixture was allowed to react for 20 hours at room temperature after which the resin was washed: 3x4 ml dimethylformamide, 3x4 ml water, 3x4 ml methanol, and 3x4 ml ether. The resin was dried in vacuo and analyzed for Fmoc-substitution. The resin product was found to contain 0.186 mmole Fmoc-alanine/g (98.4% of theoretical). Treatment of a portion of the product with neat trifluoroacetic acid for 1.5 hours at room temperature followed by analysis of Fmoc-substitution indicated that the Fmoc-alanine thus incorporated was quantitatively cleaved.

### Example 10

### Peptide Synthesis

In order to demonstrate the peptide synthesis utility of the resins (Table 2) prepared by the methods of this invention, a 9-residue peptide (Ala-Asn-Lys-Gly-Phe-Leu-Glu-Glu-Val) was synthesized starting with the Fmoc-Val-linker-Pepsyn K resin prepared as previously described. As a control for comparison, the same peptide was synthesized starting with commercially available Fmoc-Val-linker-Pepsyn K resin (Cambridge Research Biochemicals Ltd., Cambridge, England) prepared by prior art methods. (Atherton, E. et al., J.C.S. Chem. Commun. 336-337 [1981]). Both syntheses were performed on the MilliGen/ Biosearch Model 9050 continuous flow automated peptide synthesizer using commercially available (MilliGen/ Biosearch, Burlington, MA) Fmoc-amino acid-pentafluorophenyl esters as preactivated monomers and standard (default) protocols specified by MilliGen/ Biosearch Express™ peptide synthesis software (Version 1.2). Both syntheses were performed simultaneously on the same instrument using two separate column reactors such that the same reagents or solvents were simultaneously used in both syntheses. Both peptides were deprotected and isolated. Both syntheses produced the target peptide in >80% crude yield and with comparable high purities as evidenced by high performance liquid chromatography (HPLC) chromatograms (Figure 3). Figure 3a shows a chromatogram for the 9-residue peptide prepared using the solid support bound amino acid of this invention. Figure 3b show a chromatogram for a similar 9-residue peptide prepared using a commercially available solid support bound amino acid resin. Separations were performed using a Delta-Pak™ C-18 column (100A pore, 5um, 0.39x15 cm) and a flow rate of 1.0 ml/minute. Products were detected by absorbance at 220 nm. Gradient elution: (Eluent A, 0.1% (v/v) trifluoracetic acid in water; Eluent B, 0.1% (v/v) trifluoroacetic acid in 95% acetonitrile containing 5% water) 6% to 62% B in 30 minutes linearly. The identity of both products was established by amino acid analysis and mass spectral data. (M+= 1005±2, 1006 calculated)

## Claims

1. A compound of the formula: 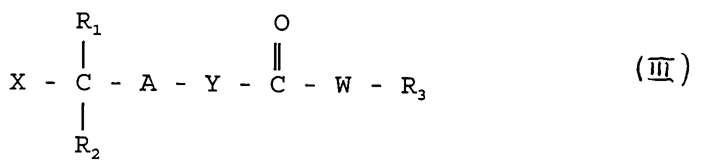
wherein X is a leaving group other than Cl;
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated sky, aryl or aralkyl which may be linked to A through an ether linkage;
W is a sulfur or oxygen atom; and
R₃ is a group derived from an alcohol, phenol, thiol, thiophenol, or heterocyclic residue.

2. A compound of Claim 1, wherein
R₃ is selected from the group consisting of dichlorophenyl, pentafluorophenyl, pentachlorophenyl, 2-nitrophenyl, 2,4-dinitrophenyl, 4-nitrophenyl, 2-chlorophenyl, 2,4,5-trichlorophenyl, 2-bromophenyl, 4-bromophenyl, trichloroethyl, phenacyl and heterocycle of 3-12 carbon atoms containing one or more nitrogen, sulfur or oxygen atoms or combinations thereof;
X is a halogen atom; and
Y is -(O)ₘ-(CH₂)ₙ - wherein m is zero or one and n is an integer from above zero to twelve.

3. A compound of Claim 1, having the formula: 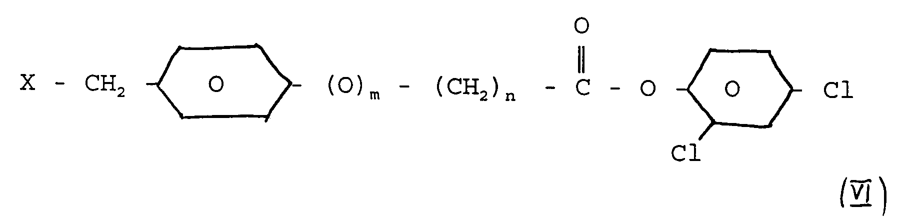 wherein X is a halogen atom other than Cl; m is zero or one and n is an integer from above zero to 12.

4. An enantiomerically pure compound of the formula: 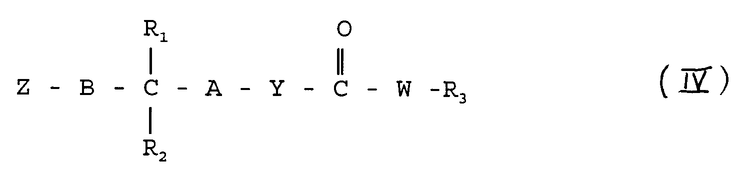
wherein Z is an Nα amino acid protecting group other than dithiosuccinyl or (isopropyldithio)carbonyl;
B is a naturally occurring D or L amino acid having protected or unprotected side chains;
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage;
W is a sulfur or oxygen atom; and
R₃ is a group derived from an alcohol, phenol, thiol, thiophenol, or heterocyclic residue.

5. A compound of Claim 4, wherein
R₃ is selected from the group consisting of dichlorophenyl, pentafluorophenyl, pentachlorophenyl, 2-nitrophenyl, 2,4-dinitrophenyl 4-nitrophenyl, 2-chlorophenyl, 2-bromophenyl, 4-bromophenyl, trichloroethyl, phenacyl and heterocycle of 3-12 carbon atoms containing one or more nitrogen, sulfur or oxygen atoms or combination thereof; and
Y is -(O)ₘ(CH₂)ₙ- where m is zero or one and n is an integer from above zero to twelve.

6. A compound of Claim 4 wherein the Nα amino acid protecting group and amino acid side chain protecting group are independently selected from the group consisting of 9-fluorenylmethyloxycarbonyl, t-butyloxycarbonyl, t-butyl, trityl, 4-methoxy-2,3,6-trimethylbenzenesulfonyl, 4,4'-dimethoxybenzhydryl, thio-t-butyl acetamidomethyl, carbobenzyloxycarbonyl, 2-nitropyridinesulfenyl, trifluoroacetyl, 2(p-biphenylisopropyloxycarbonyl), 4-methoxybenzyloxycarbonyl, tosyl, 9-phenylfluorenyl, 2-nitrophenylsulfenyl, benzyl, p-nitrocarbobenzyloxycarbonyl, t-amyloxycarbonyl, 2,2-[bis(4-nitrophenyl)]-ethyloxycarbonyl, adamantyloxycarbonyl, 2,2,2-trichloroethyloxycarbonyl, dinitrophenyl, t-butyloxymethyl, benzyloxymethyl, trichloroethyl, nitrobenzyl, phenacyl, 2,2,5,7,8-pentamethylchroman-6-sulfonyl, benzydrylamine, methylbenzydrylamine, xanthyl and 2,4,6-trimethoxybenzyl.

7. A compound of Claim 4, having the formula: 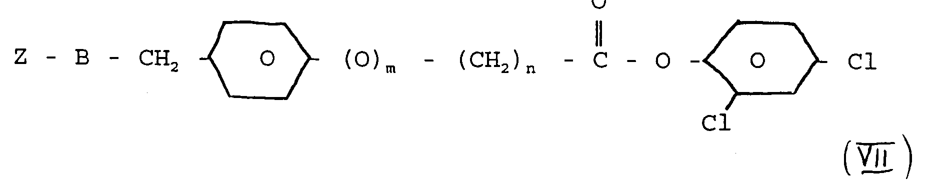
wherein Z is an Nα amino acid protecting group other than dithiosuccinyl or (isopropyldithio)carbonyl;
B is a naturally occurring D or L amino acid having protected or unprotected side chains; and
m is zero or one and n is an integer from above zero to 12.

8. A compound of Claim 7 wherein the Nα amino acid protecting group and amino acid side chain protecting group are independently selected from the group consisting of 9-fluorenylmethyloxycarbonyl, t-butyloxycarbonyl, t-butyl, trityl, thio-t-butyl, 4-methoxy-2,3,6-trimethylbenzenesulfonyl, 4,4 -dimethoxybenzhydryl, acetamidomethyl, carbobenzyloxycarbonyl, 2-nitropyridinesulfenyl, trifluoroacetyl, 2(p-biphenylisopropyloxycarbonyl), 4-methoxybenzyloxycarbonyl, tosyl, 9-phenylfluorenyl, 2-nitrophenylsulfenyl, benzyl, p-nitrocarbobenzyloxycarbonyl, t-amyloxycarbonyl, 2,2-[bis(4-nitrophenyl)]-ethyloxycarbonyl, adamantyloxycarbonyl, 2,2,2-trichloroethyloxycarbonyl, dinitrophenyl, t-butyloxymethyl, benzyloxymethyl, trichloroethyl, nitrobenzyl, phenacyl, 2,2,5,7,8-pentamethylchroman-6-sulfonyl, benzydrylamine, methylbenzydrylamine, xanthyl and 2,4,6-trimethoxybenzyl.

9. A compound of the formula: 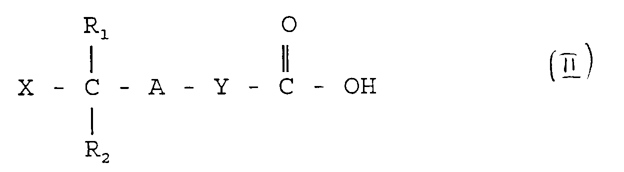
wherein X is a leaving group other than Cl;
R₁ and R₂ are the same or different and are selected from the group consisting of hydrogen, alkyl, aryl and benzyl;
A is a substituted or unsubstituted phenyl ring wherein the substituents are selected from the group consisting of aryl, alkyl, halo, nitro, alkoxy and combinations thereof; and
Y is a hydrocarbon chain having a linear or branched, saturated or unsaturated, aryl or aralkyl which may be linked to A through an ether linkage.

10. A compound of Claim 9, wherein
X is a halogen other than Cl; and
Y is -(O)ₘ(CH₂)ₙ- where m is zero or one and n is an integer from above zero to twelve.

11. A compound of Claim 9 having the formula: 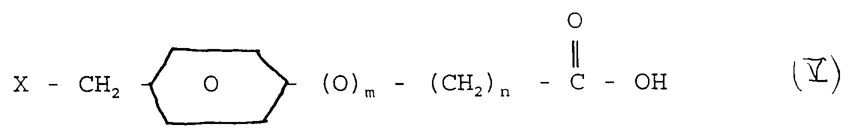 wherein X is a halogen other than Cl, m is zero or one and n is an integer from above zero to 12.

12. A compound of Claim 11 wherein the formula is 4-bromomethyl phenoxyacetic acid or 4-iodomethyl phenoxyacetic acid.

13. A method of preparing a compound of the formula: 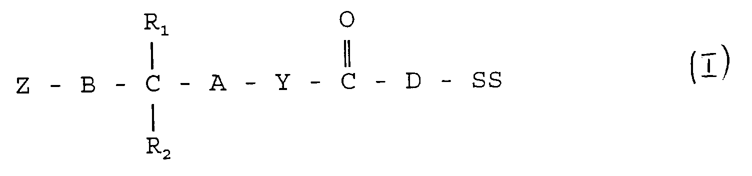
wherein Z is an Nα amino acid protecting group other than dithiosuccinyl or (isopropyldithio)carbonyl;
B is a naturally occurring D or L amino acid having protected or unprotected side chains;
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated sky, aryl or aralkyl which may be linked to A through an ether linkage;
D is an oxygen atom or -NH- which may be linked to a spacer selected from the group consisting of a natural or unnatural amino acid and a hydrocarbon chain having a linear or branched, saturated or unsaturated sky, aryl or aralkyl group, the spacer being linked to the carbonyl through an oxygen or -NH- linkage; and
SS is a solid support which is bound to the carbonyl group through D and is selected from the group consisting of polystyrene crosslinked with divinylbenzene, crosslinked polydimethylacrylamide, glass, controlled pore glass, polyvinylidinedifluoride, polypropylene, polyethylene, ceramics, silicas and aluminas; comprising the steps of:
a. providing a compound having the formula: 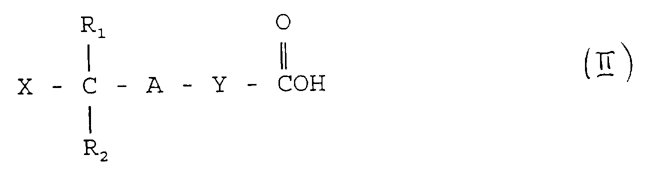 wherein X is a leaving group other than Cl and A, R₁, R₂ and Y are defined above;
b. reacting the compound of step (a) with an alcohol, phenol, thiol or thiophenol in the presence of a condensing agent to form a condensation product of the formula: 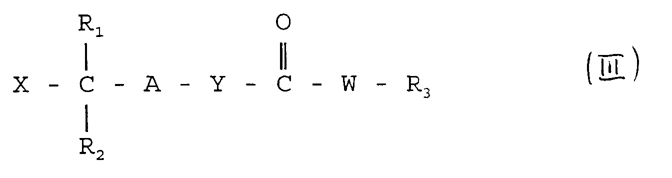
wherein X, R₁, R₂, and Y are defined above;
W is a sulfur or oxygen atom; and
R₃ is a group derived from the alcohol, phenol, thiol or thiophenol or analogous heterocyclic alcohol or thiol;
c. reacting the product of step (b) with a protected amino acid under conditions sufficient to displace X and bind the amino acid to the compound through an ester linkage; and
d. reacting the product of step (c) with a functionalized solid support under conditions suitable to remove WR₃ and couple with the functionalized solid support.

14. A method of Claim 13, wherein the alcohol, phenol, thiol or thiophenol is selected from the group consisting of substituted phenols, 2,4-dichlorophenol, trichloroethanol, phenacyl alcohol, N-hydroxysuccinimide, hydroxyl substituted heterocycles and substituted thiols.

15. The method of Claim 13 wherein the compound prepared has the formula: 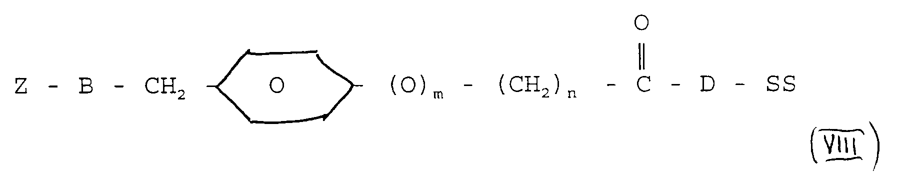
wherein Z is an Nα amino acid protecting group other than dithiosuccinyl or (isopropyldithio)carbonyl;
B is a naturally occurring D or L amino acid having a protected or unprotected side chain;
D is an oxygen atom or -NH- which may be linked to a spacer selected from the group consisting of one or more natural and unnatural amino acids or hydrocarbon chain having a linear or branched, saturated or unsaturated alkyl, aryl or aralkyl group, the spacer being linked to the carbonyl through an oxygen or -NH- linkage; and
SS is a solid support is selected from the group consisting of polystyrene crosslinked with divinylbenzene, crosslinked polydimethylacrylamide, glass, controlled pore glass, polyvinylidinedifluoride, polypropylene, polyethylene, ceramics, silicas and aluminas, which is bound to the carbonyl group through D;
wherein m is zero or one and n is an integer from above zero to 12.

16. A method of preparing a compound of the formula: 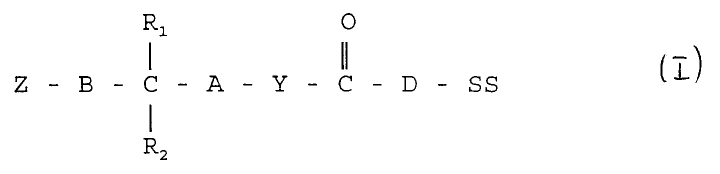
wherein Z is an Nα amino acid protecting group other than dithiosuccinyl or (isopropyldithio)carbonyl;
B is a naturally occurring D or L amino acid having protected or unprotected side chains;
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage;
D is an oxygen atom or -NH- which may be linked to a spacer selected from the group consisting of one or more natural and unnatural amino acids or hydrocarbon chain having a linear or branched, saturated or unsaturated alkyl, aryl or aralkyl group, the spacer being linked to the carbonyl through an oxygen or -NH- linkage; and
SS is a solid support which is bound to the carbonyl group through D and is selected from the group consisting of polystyrene crosslinked with divinylbenzene, crosslinked polydimethylacrylamide, glass, controlled pore glass, polyvinylidinedifluoride, polypropylene, polyethylene, ceramics, silicas and aluminas; comprising the steps of:
a. providing an aromatic carboxylic acid precursor having the formula: 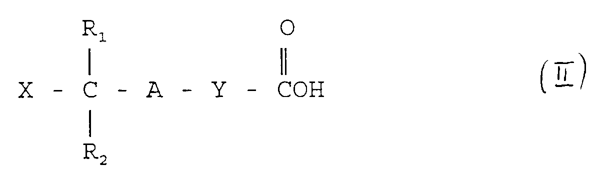 wherein X is a leaving group other than Cl and R₁, R₂, A, and Y are defined above;
b. reacting the compound of step (a) with a hydroxide source under conditions sufficient to replace X with a hydroxyl group;
c. condensing a functionalized solid support to the product of step (b);
d. reacting the product of step (c) with a reagent under conditions sufficient to replace the hydroxyl group with a leaving group; and
e. reacting the product of step (d) with a protected amino acid other than a protected amino acid caesium salt under conditions sufficient to displace the leaving group and couple the amino acid to the compound through an ester linkage in a racemization-free manner.

17. The method of Claim 16 wherein the compound prepared has the formula: 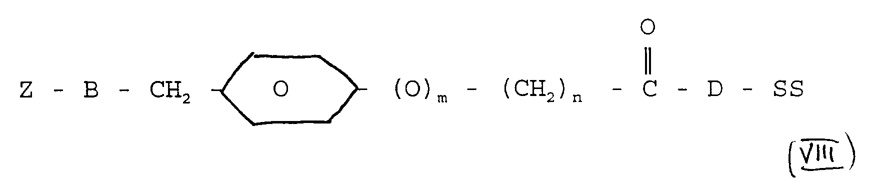
wherein Z is an Nα amino acid protecting group other than dithiosuccinyl or (isopropyldithio)carbonyl;
B is a naturally occurring D or L amino acid having a protected or unprotected side chain;
D is an oxygen atom or -NH- which may be linked to a spacer selected from the group consisting of one or more natural and unnatural amino acids or hydrocarbon chain having a linear or branched, saturated or unsaturated alkyl, aryl or aralkyl group, the spacer being linked to the carbonyl through an oxygen or -NH- linkage; and
SS is a solid support which is bound to the carbonyl group through D;
wherein m is zero or one, and n is an integer from above zero to 12.

18. A method of preparing a compound of the formula: 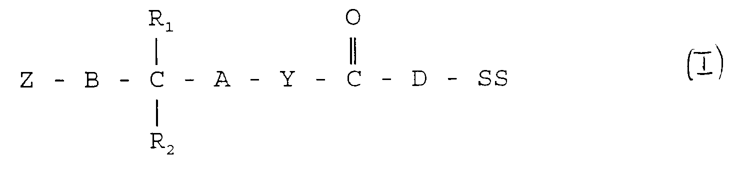
wherein Z is an Nα amino acid protecting group other than dithiosuccinyl or (isopropyldithio)carbonyl.
B is a naturally occurring D or L amino acid having protected or unprotected side chains;
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage;
D is an oxygen atom or -NH- which may be linked to a spacer selected from the group consisting of one or more natural and unnatural amino acids [or hydrocarbon chain having a linear or branched, saturated or unsaturated alkyl, aryl or aralkyl group, the spacer being linked to the carbonyl through an oxygen or -NH- linkage; and
SS is a solid support which is bound to the carbonyl group through D and is selected from the group consisting of polystyrene crosslinked with divinylbenzene, crosslinked polydimethylacrylamide, glass, controlled pore glass, polyvinylidinedifluoride, polypropylene, polyethylene, ceramics, silicas and aluminas; comprising the steps of:
a. providing a solid support bound compound having the formula: 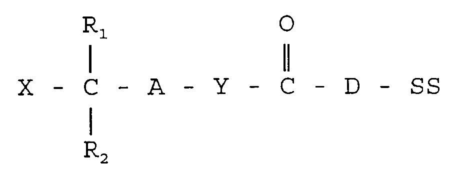 wherein X is a leaving group other than Cl and R₁, R₂, A, Y, D, and SS are defined above; and
b. reacting the compound of step (a) with a protected amino acid other than a protected amino acid caesium salt under conditions sufficient to displace the leaving group and couple the amino acid to the compound through an ester linkage in a racemization-free manner.

19. A method of synthesizing a peptide comprising the steps of:
a. providing an amino acid bound to a solid support of the formula: 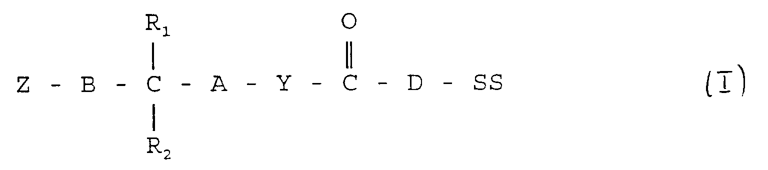
wherein Z is an Nα amino acid protecting group other than dithiosuccinyl or (isopropyldithio)carbonyl;
B is a naturally occurring D or L amino acid having protected or unprotected side chains;
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage;
D is an oxygen atom or -NH- which may be linked to a spacer selected from the group consisting of one or more natural and unnatural amino acids or hydrocarbon chain having a linear or branched, saturated or unsaturated alkyl, aryl or aralkyl group, the spacer being linked to the carbonyl through an oxygen or -NH- linkage;
SS is a solid support which is bound to the carbonyl group through D and is selected from the group consisting of polystyrene crosslinked with divinylbenzene, crosslinked polydimethylacrylamide, glass, controlled pore glass, polyvinylidinedifluoride, polypropylene, polyethylene, ceramics, silicas and aluminas; and
b. sequentially coupling protected amino acids other than protected amino acid caesium salts to B to produce a solid support bound peptide.

20. A method of Claim 19, further comprising the step of removing the protecting groups from the solid support bound peptide.

21. A method of Claim 19 wherein the synthesized peptide is cleaved from the solid support.

22. A method according to Claim 19 wherein the amino acid bound to a solid support has the formula: 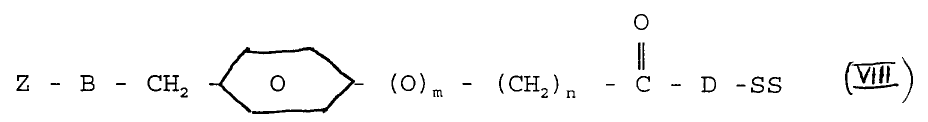
wherein Z is an Nα amino acid protecting group other than dithiosuccinyl or (isopropyldithio) carbonyl;
B is a naturally occurring D or L amino acid having a protected or unprotected side chain;
m is zero or one and n is an integer from above zero to 12;
D is an oxygen atom or -NH- which may be linked to a spacer selected from the group consisting of a natural or unnatural amino acid and a hydrocarbon chain having a linear or branched, saturated or unsaturated alkyl, aryl or aralkyl group, the spacer being linked to the carbonyl through an oxygen or -NH- linkage; and
SS is a solid support which is bound to the carbonyl group through D and is selected from the group consisting of polystyrene crosslinked with divinylbenzene, crosslinked polydimethylacrylamide, glass, controlled pore glass, polyvinylidinedifluoride, polypropylene, polyethylene, ceramics, silicas and aluminas.

23. A method of preparing a compound of Claim 19, comprising the steps of:
a. providing a compound having a benzylic hydrogen of the formula: 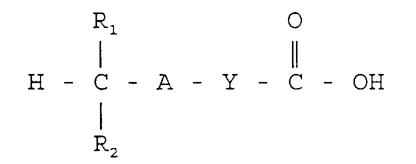
wherein R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage; and
b. reacting the compound of step (a) with a reagent under conditions suitable to replace the benzylic hydrogen with the leaving group.

24. A method of preparing a compound of Claim 1, comprising the steps of:
a. providing a compound having the formula: 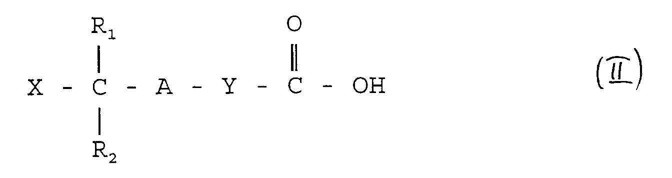
wherein X is a leaving group other than Cl;
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage; and
b. reacting the compound of step (a) with an alcohol, phenol, thiol or thiophenol which may be heterocyclic, in the presence of a condensing agent under conditions suitable to produce the above compound of Claim 1 where WR₃ is a group analogous to the alcohol, phenol, thiol or thiophenol.

25. A method of Claim 24 wherein the alcohol, phenol, thiol or thiophenol is selected from the group consisting of substituted phenols, 2,4-dichlorophenol, trichloroethanol, phenacyl alcohol, N-hydroxysuccinimide, hydroxyl substituted heterocycles and substituted thiols.

26. A method of Claim 25, further comprising the step:
c. replacing X with a protected amino acid under conditions suitable to bind the amino acid to the compound through an ester linkage.

27. A method of preparing a compound of the formula: 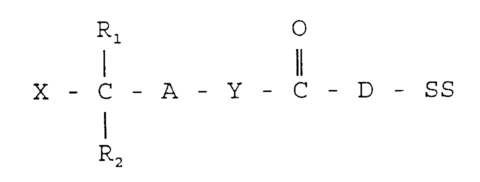
wherein X is a leaving group other than Cl:
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage;
D is an oxygen atom or -NH- which may be linked to a spacer selected from the group consisting of one or more natural and unnatural amino acids or hydrocarbon chain having a linear or branched, saturated or unsaturated alkyl, aryl or aralkyl group, the spacer being linked to the carbonyl through an oxygen or -NH- linkage; and
SS is a solid support which is bound to the carbonyl group through D and is selected from the group consisting of polystyrene crosslinked with divinylbenzene, crosslinked polydimethylacrylamide, glass, controlled pore glass, polyvinylidinedifluoride, polypropylene, polyethylene, ceramics, silicas and aluminas; comprising the steps of:
a. providing a compound having the formula: 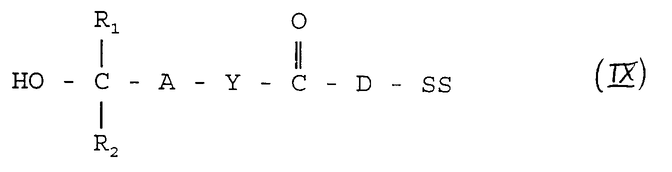 wherein R₁, R₂, A, Y, D and SS are defined above; and
b. reacting the compound of step (a) with a reagent under conditions sufficient to displace the hydroxyl group with a leaving group X.

28. A method of Claim 27 wherein the compound prepared has the formula: 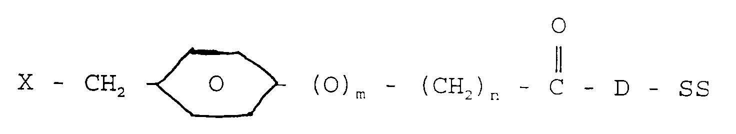
wherein X is a halogen other than Cl;
m is zero or one and n is an integer from above zero to 12.

29. A method of Claim 27 further comprising the step:
c. reacting the compound of step b with a protected amino acid under conditions sufficient to displace the leaving group and couple the amino acid to the compound through an ester linkage in a racemization-free manner.

30. A method of preparing a compound having the formula: 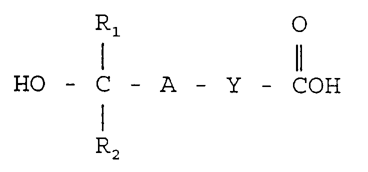
R₁ and R₂ are the same or different and are selected from the group consisting of hydrogen, linear or branched, saturated or unsaturated alkyl, aryl and benzyl;
A is a substituted or unsubstituted phenyl ring wherein the substituents are selected from the group consisting of aryl, alkyl, halo, nitro, alkoxy and combinations thereof;
Y is a hydrocarbon chain having a linear or branched, saturated or unsaturated alkyl, aryl or aralkyl which may be linked to A through an ether linkage, comprising the steps of:
a. providing a compound having the formula: 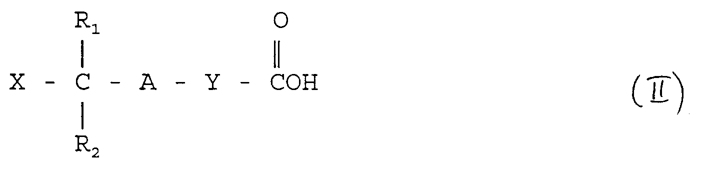 wherein X is a leaving group other than Cl and R₁, R₂, A and Y are defined above; and
b. reacting the compound of step (a) with a hydroxide source under conditions sufficient to replace X with a hydroxyl group.

31. A method of Claim 30 wherein the compound of step (a) is 4-bromomethylphenoxyacetic acid.

## Patentansprüche

1. Verbindung der Formel: 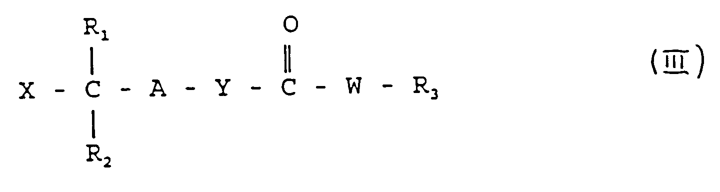 worin bedeuten:
X eine von Cl verschiedene, abspaltbare Gruppe bzw. flüchtige Gruppe;
R₁ und R₂, die gleich oder verschieden sind, Wasserstoff, lineares oder verzweigtkettiges, gesättigtes oder ungesättigtes Alkyl, Aryl oder Benzyl;
A einen gegebenenfalls substituierten Phenylring, wobe die Substituenten aus der Gruppe Aryl, Alkyl, Halogen, Nitro, Alkoxy und Kombinationen derselben ausgewählt sind;
Y eine Kohlenwasserstoffkette mit linearem oder verzweigtkettigem, gesättigtem oder ungesättigtem Alkyl, Aryl oder Aralkyl, das an A über eine Etherbindung gebunden sein kann;
W ein Schwefel- oder Sauerstoffatom, und
R₃ eine von einem Alkohol, Phenol, Thiol, Thiophenol oder heterocyclischen Rest herrührende Gruppe.

2. Verbindung nach Anspruch 1, wobei R₃ aus der Gruppe Dichlorphenyl, Pentafluorphenyl, Pentachlorphenyl, 2-Nitrophenyl, 2,4-Dinitrophenyl, 4-Nitrophenyl, 2-Chlorphenyl, 2,4,5-Trichlorphenyl, 2-Bromphenyl, 4-Bromphenyl, Trichlorethyl, Phenacyl und einem Heterocyclus mit 3 bis 12 Kohlenstoffatomen mit einem oder mehreren Stickstoff-, Schwefel- oder Sauerstoffatom(en) oder Kombinationen derselben ausgewählt ist;
X ein Halogenatom darstellt und
Y für -(O)ₘ-(CH₂)ₙ- mit m = 0 oder 1 und n einer ganzen Zahl über 0 bis 12 steht.

3. Verbindung nach Anspruch 1 der Formel: 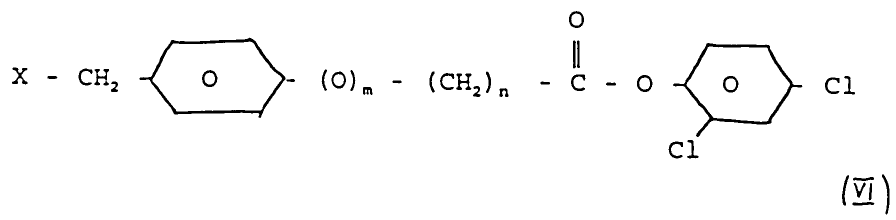 worin bedeuten:
X ein von Cl verschiedenes Halogenatom;
m = 0 oder 1 und
n eine ganze Zahl von über 0 bis 12.

4. Enantiomerenreine Verbindung der Formel: 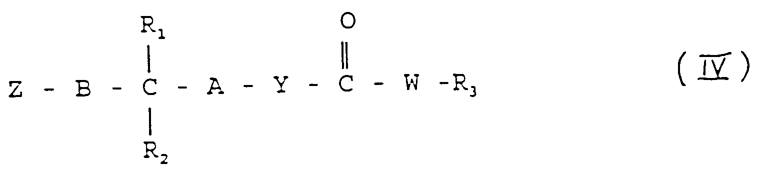 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützten oder ungeschützten Seitenketten;
R₁ und R₂, die gleich oder verschieden sind, Wasserstoff, lineares oder verzweigtkettiges, gesättigtes oder ungesättigtes Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind;
Y eine Kohlenwasserstoffkette mit linearem oder verzweigtkettigem, gesättigtem oder ungesättigtem Alkyl, Aryl oder Aralkyl, das an A über eine Etherbindung gebunden sein kann;
W ein Schwefel- oder Sauerstoffatom, und
R₃ eine von einem Alkohol, Phenol, Thiol, Thiophenol oder heterocyclischen Rest herrührende Gruppe.

5. Verbindung nach Anspruch 4, wobei R₃ aus der Gruppe Dichlorphenyl, Pentafluorphenyl, Pentachlorphenyl, 2-Nitrophenyl, 2,4-Dinitrophenyl, 4-Nitrophenyl, 2-Chlorphenyl, 2-Bromphenyl, 4-Bromphenyl, Trichlorethyl, Phenacyl und einem Heterocyclus mit 3 bis 12 Kohlenstoffatomen mit einem oder mehreren Stickstoff-, Schwefel- oder Sauerstoffatom(en) oder Kombinationen derselben ausgewählt ist, und
Y -(O)ₘ-(CH₂)ₙ- mit m = 0 oder 1 und n einer ganzen Zahl über 0 bis 12 bedeutet.

6. Verbindung nach Anspruch 4, wobei die Nα-Aminosäureschutzgruppe und Aminosäureseitenkettenschutzgruppe unabhängig voneinander aus der Gruppe 9-Fluorenylmethyloxycarbonyl, tert.-Butyloxycarbonyl, tert.-Butyl, Trityl, 4-Methoxy-2,3,6-trimethylbenzolsulfonyl, 4,4'-Dimethoxybenzhydryl, Thio-tert.-butylacetamidomethyl, Carbobenzyloxycarbonyl, 2-Nitropyridinsulfenyl, Trifluoracetyl, 2(p-Biphenylisopropyloxycarbonyl), 4-Methoxybenzyloxycarbonyl, Tosyl, 9-Phenylfluorenyl, 2-Nitrophenylsulfenyl, Benzyl, p-Nitrocarbobenzyloxycarbonyl, tert.-Amyloxycarbonyl, 2,2-[Bis(4-nitrophenyl)]ethyloxycarbonyl, Adamantyloxycarbonyl, 2,2,2-Trichlorethyloxycarbonyl, Dinitrophenyl, tert.-Butyloxymethyl, Benzyloxymethyl, Trichlorethyl, Nitrobenzyl, Phenacyl, 2,2,5,7,8-Pentamethylchroman-6-sulfonyl, Benzydrylamin, Methylbenzydrylamin, Xanthyl und 2,4,6-Trimethoxybenzyl ausgewählt sind.

7. Verbindung nach Anspruch 4 der Formel 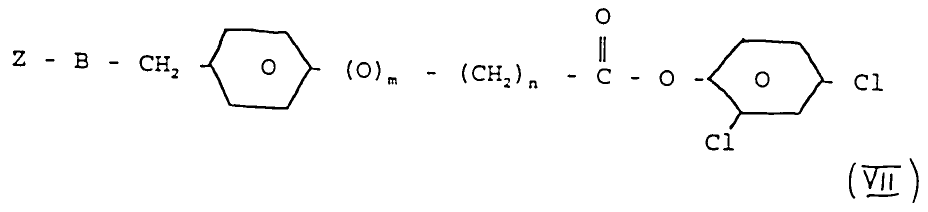 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützten oder ungeschützten Seitenketten;
m = 0 oder 1 und
n eine ganze Zahl von über 0 bis 12.

8. Verbindung nach Anspruch 7, wobei die Nα-Aminosäureschutzgruppe und Aminosäureseitenkettenschutzgruppe unabhängig voneinander aus der Gruppe 9-Fluorenylmethyloxycarbonyl, tert.-Butyloxycarbonyl, tert.-Butyl, Trityl, Thio-tert.-butyl, 4-Methoxy-2,3,6-trimethylbenzolsulfonyl, 4,4'-Dimethoxybenzhydryl, Acetamidomethyl, Carbobenzyloxycarbonyl, 2-Nitropyridinsulfenyl, Trifluoracetyl, 2(p-Biphenylisopropyloxycarbonyl), 4-Methoxybenzyloxycarbonyl, Tosyl, 9-Phenylfluorenyl, 2-Nitrophenylsulfenyl, Benzyl, p-Nitrocarbobenzyloxycarbonyl, tert.-Amyloxycarbonyl, 2,2-[Bis(4-nitrophenyl)]ethyloxycarbonyl, Adamantyloxycarbonyl, 2,2,2-Trichlorethyloxycarbonyl, Dinitrophenyl, tert.-Butyloxymethyl, Benzyloxymethyl, Trichlorethyl, Nitrobenzyl, Phenacyl, 2,2,5,7,8-Pentamethylchroman-6-sulfonyl, Benzydrylamin, Methylbenzydrylamin, Xanthyl und 2,4,6-Trimethoxybenzyl ausgewählt sind.

9. Verbindung der Formel: 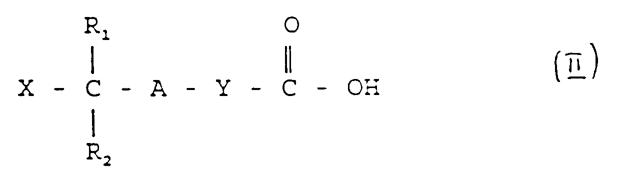 worin bedeuten:
X eine von Cl verschiedene abspaltbare bzw. flüchtige Gruppe;
R₁ und R₂, die gleich oder verschieden sind, Wasserstoff, Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind, und
Y eine Kohlenwasserstoffkette mit linearem oder verzweigtkettigem, gesättigtem oder ungesättigtem Aryl oder Aralkyl, das an A über eine Etherbindung gebunden sein kann.

10. Verbindung nach Anspruch 9, worin bedeuten:
X ein von Cl verschiedenes Halogen, und
Y -(O)ₘ-(CH₂)ₙ- mit m = 0 oder 1 und n einer ganzen Zahl über 0 bis 12.

11. Verbindung nach Anspruch 9 der Formel: 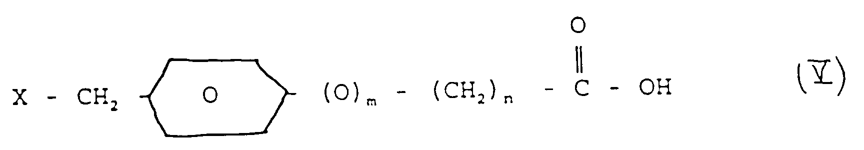 worin bedeuten:
X ein von Cl verschiedenes Halogen;
m = 0 oder 1 und
n eine ganze Zahl von über 0 bis 12.

12. Verbindung nach Anspruch 11, nämlich 4-Brommethylphenoxyessigsäure oder 4-Iodmethylphenoxyessigsäure.

13. Verfahren zur Herstellung einer Verbindung der Formel: 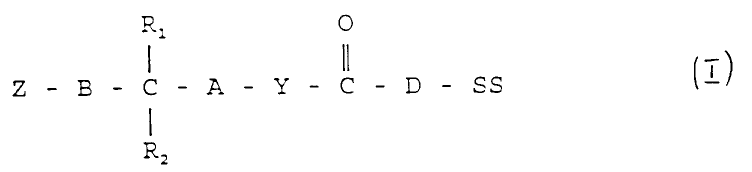 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützten oder ungeschützten Seitenketten;
R₁ und R₂, die gleich oder verschieden sind, Wasserstoff, lineares oder verzweigtkettiges, gesättigtes oder ungesättigtes Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind;
Y eine Kohlenwasserstoffkette mit linearem oder verzweigtkettigem, gesättigtem oder ungesättigtem Alkyl, Aryl oder Aralkyl, das an A über eine Etherbindung gebunden sein kann;
D ein Sauerstoffatom oder -NH-, das an einen Spacer, ausgewählt aus der Gruppe natürliche oder unnatürliche Aminosäure- und Kohlenwasserstoffketten mit einer linearen oder verzweigtkettigen, gesättigten oder ungesättigten Alkyl-, Aryl- oder Aralkylgruppe, gebunden sein kann, wobei der Spacer an das Carbonyl über eine Sauerstoff- oder -NH-Bindung gebunden ist, und
SS einen festen Träger, der an die Carbonylgruppe über D gebunden und aus der Gruppe mit Divinylbenzol vernetztes Polystyrol, vernetztes Polydimethylacrylamid, Glas, porengesteuertes Glas, Polyvinylidendifluorid, Polypropylen, Polyethylen, Keramiken, Siliciumdioxide und Aluminiumoxide ausgewählt ist,
in folgenden Stufen:
a. Bereitstellen einer Verbindung der Formel: 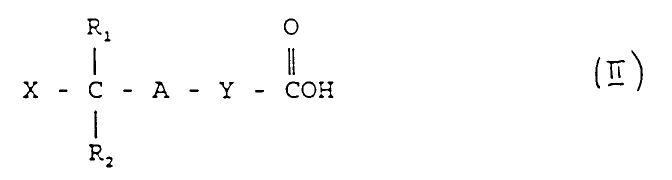 worin X für eine von Cl verschiedene abspaltbare bzw. flüchtige Gruppe steht und A, R₁, R₂ und Y die zuvor angegebene Bedeutung besitzen;
b. Umsetzen der Verbindung aus Stufe (a) mit einem Alkohol, Phenol, Thiol oder Thiophenol in Gegenwart eines Kondensationsmittels zur Bildung eines Kondensationsprodukts der Formel: 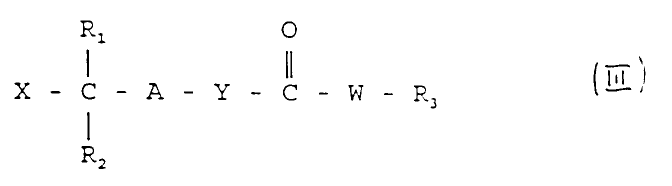
worin X, R₁, R₂ und Y die zuvor angegebene Bedeutung besitzen,
W für ein Schwefel- oder Sauerstoffatom steht und
R₃ eine von einem Alkohol, Phenol, Thiol oder Thiophenol oder analogen heterocyclischen Alkohol oder Thiol herrührende Gruppe bedeutet;
c. Umsetzen des Produkts aus Stufe (b) mit einer geschützten Aminosäure unter Bedingungen, die für eine Verdrängung von X und zur Bindung der Aminosäure an die Verbindung über eine Esterbindung ausreichen, und
d. Umsetzen des Produkts aus Stufe (c) mit einem funktionalisierten festen Träger unter Bedingungen, die für eine Entfernung von WR₃ und Kupplung mit dem funktionalisierten festen Träger ausreichend sind.

14. Verfahren nach Anspruch 13, wobei der Alkohol, das Phenol, das Thiol oder das Thiophenol aus der Gruppe substituierte Phenole, 2,4-Dichlorphenol, Trichlorethanol, Phenacylalkohol, N-Hydroxysuccinimid, hydroxylsubstituierte Heterocyclen und substituierte Thiole ausgewählt ist.

15. Verfahren nach Anspruch 13, wobei die erhaltene Verbindung der Formel: 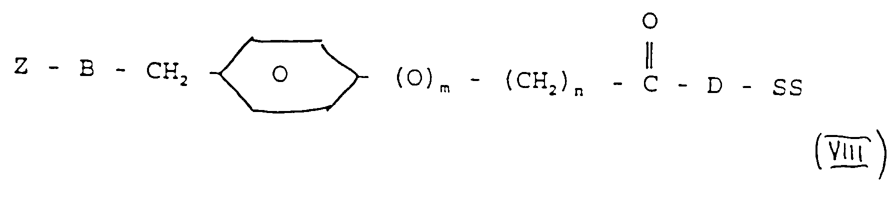 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützter oder ungeschützter Seitenkette;
D ein Sauerstoffatom oder -NH-, das an einen Spacer, ausgewählt aus der Gruppe eine oder mehrere natürliche oder unnatürliche Aminosäure- und Kohlenwasserstoffkette(n) mit einer linearen oder verzweigtkettigen, gesättigten oder ungesättigten Alkyl-, Aryl- oder Aralkylgruppe, gebunden sein kann, wobei der Spacer an das Carbonyl über eine Sauerstoff- oder -NH-Bindung gebunden ist, und
SS einen festen Schichtträger, der aus der Gruppe mit Divinylbenzol vernetztes Polystyrol, vernetztes Polydimethylacrylamid, Glas, porengesteuertes Glas, Polyvinylidendifluorid, Polypropylen, Polyethylen, Keramiken, Siliciumdioxide und Aluminiumoxide ausgewählt und über D an die Carbonylgruppe gebunden ist;
m = 0 oder 1, und n eine ganze Zahl von über 0 bis 12
entspricht.

16. Verfahren zur Herstellung einer Verbindung der Formel: 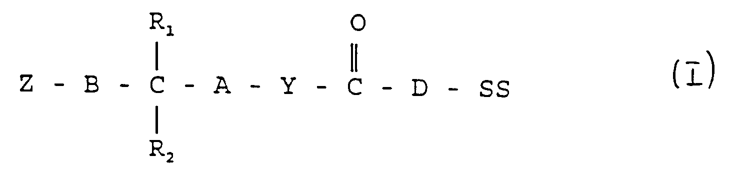 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützten oder ungeschützten Seitenketten;
R₁ und R₂, die gleich oder verschieden sind, Wasserstoff, lineares oder verzweigtkettiges, gesättigtes oder ungesättigtes Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind;
Y eine Kohlenwasserstoffkette mit linearem oder verzweigtkettigem, gesättigtem oder ungesättigtem Alkyl, Aryl oder Aralkyl, das an A über eine Etherbindung gebunden sein kann;
D ein Sauerstoffatom oder -NH-, das an einen Spacer, ausgewählt aus der Gruppe eine oder mehrere natürliche oder unnatürliche Aminosäure- und Kohlenwasserstoffkette(n) mit einer linearen oder verzweigtkettigen, gesättigten oder ungesättigten Alkyl-, Aryl- oder Aralkylgruppe, gebunden sein kann, wobei der Spacer an das Carbonyl über eine Sauerstoff- oder -NH-Bindung gebunden ist, und
SS einen festen Träger, der an die Carbonylgruppe über D gebunden und aus der Gruppe mit Divinylbenzol vernetztes Polystyrol, vernetztes Polydimethylacrylamid, Glas, porengesteuertes Glas, Polyvinylidendifluorid, Polypropylen, Polyethylen, Keramiken, Siliciumdioxide und Aluminiumoxide ausgewählt ist,
in folgenden Stufen:
a. Bereitstellen eines aromatischen Carbonsäurevorläufers der Formel: 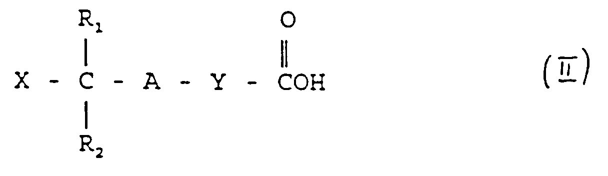 worin X für eine von Cl verschiedene abspaltbare bzw. flüchtige Gruppe steht und R₁, R₂, A und Y die zuvor angegebene Bedeutung besitzen;
b. Umsetzen der Verbindung aus Stufe (a) mit einer Hydroxidquelle unter zum Ersatz von X durch eine Hydroxylgruppe ausreichenden Bedingungen;
c. Kondensieren eines funktionalisierten festen Trägers mit dem Produkt aus Stufe (b);
d. Umsetzen des Produkts aus Stufe (c) mit einem Reagens unter zum Ersatz der Hydroxylgruppe durch eine abspaltbare bzw. flüchtige Gruppe ausreichenden Bedingungen und
e. Umsetzen des Produkts aus Stufe (d) mit einer von einem geschützten Aminosäurecäsiumsalz verschiedenen geschützten Aminosäure unter Bedingungen, die zur Verdrängung der abspaltbaren bzw. flüchtigen Gruppe und Kupplung der Aminosäure an die Verbindung über eine Esterbindung in racemisierungsfreier Art und Weise ausreichen.

17. Verfahren nach Anspruch 16, wobei der erhaltenen Verbindung die Formel: 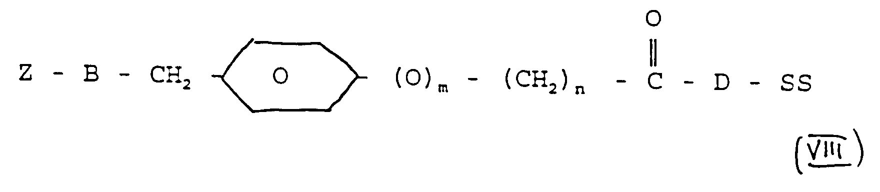 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützter oder ungeschützter Seitenkette;
D ein Sauerstoffatom oder -NH-, das an einen Spacer, ausgewählt aus der Gruppe eine oder mehrere natürliche oder unnatürliche Aminosäure- und Kohlenwasserstoffkette(n) mit einer linearen oder verzweigtkettigen, gesättigten oder ungesättigten Alkyl-, Aryl- oder Aralkylgruppe, gebunden sein kann, wobei der Spacer an das Carbonyl über eine Sauerstoff- oder -NH-Bindung gebunden ist, und
SS einen festen Träger, der über D an die Carbonylgruppe gebunden ist;
m = 0 oder 1, und
n eine ganze Zahl von über 0 bis 12 besitzt,
zukommt.

18. Verfahren zur Herstellung einer Verbindung der Formel: 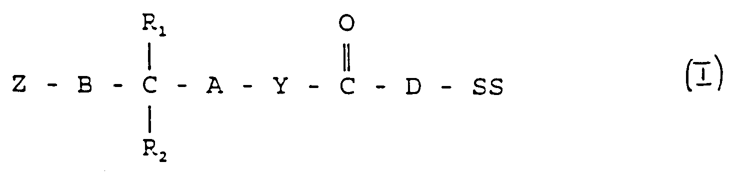 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützten oder ungeschützten Seitenketten;
R₁ und R₂, die gleich oder verschieden sind, Wasserstoff, lineares oder verzweigtkettiges, gesättigtes oder ungesättigtes Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind;
Y eine Kohlenwasserstoffkette mit linearem oder verzweigtkettigem, gesättigtem oder ungesättigtem Alkyl, Aryl oder Aralkyl, das an A über eine Etherbindung gebunden sein kann;
D ein Sauerstoffatom oder -NH-, das an einen Spacer, ausgewählt aus der Gruppe eine oder mehrere natürliche oder unnatürliche Aminosäure- und Kohlenwasserstoffkette(n) mit einer linearen oder verzweigtkettigen, gesättigten oder ungesättigten Alkyl-, Aryl- oder Aralkylgruppe, gebunden sein kann, wobei der Spacer an das Carbonyl über eine Sauerstoff- oder -NH-Bindung gebunden ist, und
SS einen festen Träger, der an die Carbonylgruppe über D gebunden und aus der Gruppe mit Divinylbenzol vernetztes Polystyrol, vernetztes Polydimethylacrylamid, Glas, porengesteuertes Glas, Polyvinylidendifluorid, Polypropylen, Polyethylen, Keramiken, Siliciumdioxide und Aluminiumoxide ausgewählt ist,
in folgenden Stufen:
a. Bereitstellen einer an einen festen Träger gebundenen Verbindung der Formel: 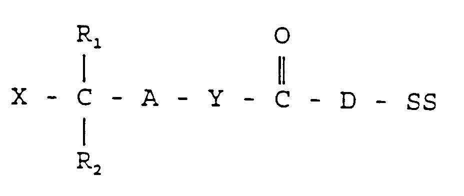 worin X für eine von Cl verschiedene abspaltbare bzw. flüchtige Gruppe steht und R₁, R₂, A, Y, D und SS die zuvor angegebene Bedeutung besitzen, und
b. Umsetzen der Verbindung aus Stufe (a) mit einer von einem geschützten Aminosäurecäsiumsalz verschiedenen geschützten Aminosäure unter Bedingungen, die zur Verdrängung der abspaltbaren bzw. flüchtigen Gruppe und Kupplung der Aminosäure an die Verbindung über eine Esterbindung in racemisierungsfreier Art und Weise ausreichen.

19. Verfahren zur Synthese eines Peptids in folgenden Stufen:
a. Bereitstellen einer an einen festen Träger gebundenen Aminosäure der Formel: 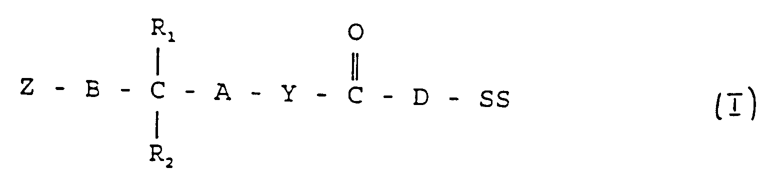 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützten oder ungeschützten Seitenketten;
R₁ und R₂, die gleich oder verschieden sind, Wasserstoff, lineares oder verzweigtkettiges, gesättigtes oder ungesattigtes Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind;
Y eine Kohlenwasserstoffkette mit linearem oder verzweigtkettigem, gesättigtem oder ungesättigtem Alkyl, Aryl oder Aralkyl, das an A über eine Etherbindung gebunden sein kann;
D ein Sauerstoffatom oder -NH-, das an einen Spacer, ausgewählt aus der Gruppe eine oder mehrere natürliche oder unnatürliche Aminosäure- und Kohlenwasserstoffkette(n) mit einer linearen oder verzweigtkettigen, gesättigten oder ungesättigten Alkyl-, Aryl- oder Aralkylgruppe, gebunden sein kann, wobei der Spacer an das Carbonyl über eine Sauerstoff- oder -NH-Bindung gebunden ist, und
SS einen festen Träger, der an die Carbonylgruppe über D gebunden und aus der Gruppe mit Divinylbenzol vernetztes Polystyrol, vernetztes Polydimethylacrylamid, Glas, porengesteuertes Glas, Polyvinylidendifluorid, Polypropylen, Polyethylen, Keramiken, Siliciumdioxide und Aluminiumoxide, und
b. sequentielles Kuppeln geschützter und von geschützten Aminosäurecäsiumsalzen verschiedenen Aminosäuren an B zur Bildung eines an einen festen Träger gebundenen Peptids.

20. Verfahren nach Anspruch 19, bei welchem zusätzlich die Schutzgruppen aus dem an einen festen Träger gebundenen Peptid entfernt werden.

21. Verfahren nach Anspruch 19, bei welchem das synthetisierte Peptid von dem festen Träger abgespalten wird.

22. Verfahren nach Anspruch 19, wobei der an einen festen Träger gebundenen Aminosäure die Formel: 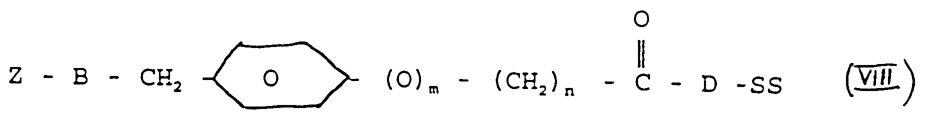 worin bedeuten:
Z eine von Dithiosuccinyl oder (Isopropyldithio)carbonyl verschiedene Nα-Aminosäureschutzgruppe;
B eine natürlich vorkommende D- oder L-Aminosäure mit geschützter oder ungeschützter Seitenkette;
m = 0 oder 1;
n eine ganze Zahl von über 0 bis 12;
D ein Sauerstoffatom oder -NH-, das an einen Spacer, ausgewählt aus der Gruppe natürliche oder unnatürliche Aminosäure- und Kohlenwasserstoffketten mit einer linearen oder verzweigtkettigen, gesättigten oder ungesättigten Alkyl-, Aryl- oder Aralkylgruppe, gebunden sein kann, wobei der Spacer an das Carbonyl über eine Sauerstoff- oder -NH-Bindung gebunden ist, und
SS einen an die Carbonylgruppe über D gebundenen festen Träger, ausgewählt aus der Gruppe mit Divinylbenzol vernetztes Polystyrol, vernetztes Polydimethylacrylamid, Glas, porengesteuertes Glas, Polyvinylidendifluorid, Polypropylen, Polyethylen, Keramiken, Siliciumdioxide und Aluminiumoxide
zukommt.

23. Verfahren zur Herstellung der Verbindung nach Anspruch 19, in folgenden Stufen:
a. Bereitstellen einer Verbindung mit einem Benzylwasserstoff der Formel: 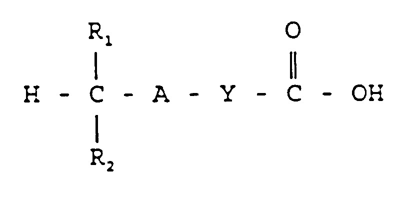 worin bedeuten:
R₁ und R₂, die gleich oder verschieden sind, Wasserstoff, lineares oder verzweigtkettiges, gesättigtes oder ungesättigtes Alkyl, Aryl oder Benzyl;
A einen gegebenenfalls substituierten Phenylring, wobei die Substituenten aus der Gruppe, Aryl, Alkyl, Halogen, Nitro, Alkoxy und Kombinationen derselben ausgewählt sind, und
Y eine Kohlenwasserstoffkette mit linearem oder verzweigtkettigem, gesättigtem oder ungesättigtem Alkyl, Aryl oder Aralkyl, das an A über eine Etherbindung gebunden sein kann, und
b. Umsetzen der Verbindung aus Stufe (a) mit einem Reagens unter zum Ersatz des Benzylwasserstoffs durch die abspaltbare bzw. flüchtige Gruppe geeigneten Bedingungen.

24. Verfahren zur Herstellung einer Verbindung nach Anspruch 1 in folgenden Stufen:
a. Bereitstellen einer Verbindung der Formel: 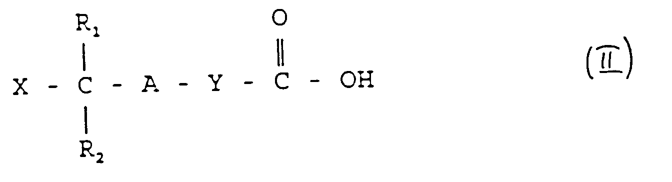 worin bedeuten:
X eine von Cl verschiedene abspaltbare bzw. flüchtige Gruppe;
R₁ und R₂, die gleich oder verschieden sind, Wasserstoff, Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind, und
Y eine Kohlenwasserstoffkette mit linearem oder verzweigtkettigem, gesättigtem oder ungesättigtem Alkyl, Aryl oder Aralkyl, das an A über eine Etherbindung gebunden sein kann, und
b. Umsetzen der Verbindung aus Stufe (a) mit einem gegebenenfalls heterocyclischen Alkohol, Phenol, Thiol oder Thiophenol in Gegenwart eines Kondensationsmittels unter Bedingungen, die zur Herstellung der genannten Verbindung nach Anspruch 1 mit WR₃ gleich einer zu dem Alkohol, Phenol, Thiol oder Thiophenol analogen Gruppe geeignet sind.

25. Verfahren nach Anspruch 24, wobei der Alkohol, das Phenol, das Thiol oder das Thiophenol aus der Gruppe substituierte Phenole, 2,4-Dichlorphenol, Trichlorethanol, Phenacylalkohol, N-Hydroxysuccinimid, hydroxylsubstituierte Heterocyclen und substituierte Thiole ausgewählt ist.

26. Verfahren nach Anspruch 25, bei welchem zusätzlich in einer Stufe (c) X durch eine geschützte Aminosäure unter Bedingungen, die zur Bindung der Aminosäure an die betreffende Verbindung über eine Esterbindung geeignet sind, ersetzt wird.

27. Verfahren zur Herstellung einer Verbindung der Formel: 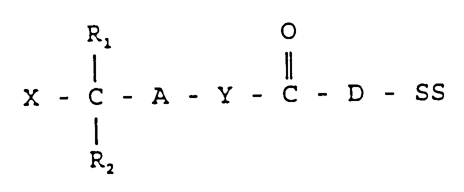 worin bedeuten:
X eine von Cl verschiedene abspaltbare bzw. flüchtige Gruppe;
R₁ und R₂, die gleich oder verschieden sind, Wasserstoff, lineares oder verzweigtkettiges, gesättigtes oder ungesättigtes Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind;
Y eine Kohlenwasserstoffkette mit linearem oder verzweigtkettigem, gesättigtem oder ungesättigtem Alkyl, Aryl oder Aralkyl, das an A über eine Etherbindung gebunden sein kann;
D ein Sauerstoffatom oder -NH-, das an einen Spacer, ausgewählt aus der Gruppe eine oder mehrere natürliche oder unnatürliche Aminosäure- und Kohlenwasserstoffkette(n) mit einer linearen oder verzweigtkettigen, gesättigten oder ungesättigten Alkyl-, Aryl- oder Aralkylgruppe, gebunden sein kann, wobei der Spacer an das Carbonyl über eine Sauerstoff- oder -NH-Bindung gebunden ist, und
SS einen festen Träger, der an die Carbonylgruppe über D gebunden und aus der Gruppe mit Divinylbenzol vernetztes Polystyrol, vernetztes Polydimethylacrylamid, Glas, porengesteuertes Glas, Polyvinylidendifluorid, Polypropylen, Polyethylen, Keramiken, Siliciumdioxide und Aluminiumoxide, in folgenden Stufen:
a. Bereitstellen einer Verbindung der Formel: 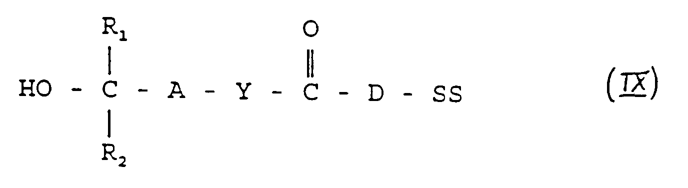 worin R₁, R₂, A, Y, D und SS die zuvor angegebene Bedeutung besitzen, und
b. Umsetzen der Verbindung aus Stufe (a) mit einem Reagens unter Bedingungen, die zur Verdrängung der Hydroxylgruppe durch eine abspaltbare bzw. flüchtige Gruppe X ausreichend sind.

28. Verfahren nach Anspruch 27, wobei der erhaltenen Verbindung die Formel: 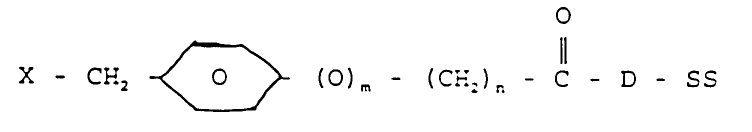 worin bedeuten:
X ein von Cl verschiedenes Halogen;
m = 0 oder 1, und
n eine ganze Zahl über 0 bis 12
zukommt.

29. Verfahren nach Anspruch 27, bei welchem zusätzlich in einer Stufe (c) die Verbindung aus Stufe (b) mit einer geschützten Aminosäure unter Bedingungen, die zur Verdrängung der abspaltbaren bzw. flüchtigen Gruppe und Kupplung der Aminosäure an die betreffende Verbindung über eine Esterbindung in racemisierungsfreier Art und Weise ausreichen, umgesetzt wird.

30. Verfahren zur Herstellung einer Verbindung der Formel: 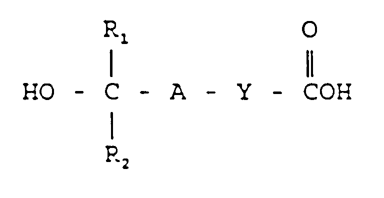 worin bedeuten:
R₁ und R₂, die gleich oder verschieden sind, Wasserstoff, lineares oder verzweigtkettiges, gesättigtes oder ungesättigtes Alkyl, Aryl oder Benzyl;
A einen gegebenenfalls substituierten Phenylring, wobei die Substituenten aus der Gruppe Aryl, Alkyl, Halogen, Nitro, Alkoxy und Kombinationen derselben ausgewählt sind, und
Y eine Kohlenwasserstoffkette mit linearem oder verzweigtkettigem, gesättigtem oder ungesättigtem Alkyl, Aryl oder Aralkyl, das an A über eine Etherbindung gebunden sein kann, in folgenden Stufen:
a. Bereitstellen einer Verbindung der Formel: 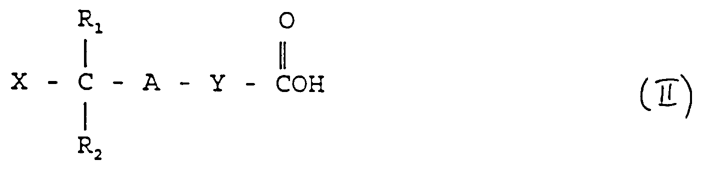 worin X für eine von Cl verschiedene abspaltbare bzw. flüchtige Gruppe steht und R₁, R₂, A und Y die zuvor angegebene Bedeutung besitzen, und
b. Umsetzen der Verbindung aus Stufe (a) mit einer Hydroxidquelle unter Bedingungen, die zur Verdrängung von X durch eine Hydroxylgruppe ausreichen.

31. Verfahren nach Anspruch 30, wobei es sich bei der Verbindung von Stufe (a) um 4-Brommethylphenoxyessigsäure handelt.

## Revendications

1. Composé de formule: 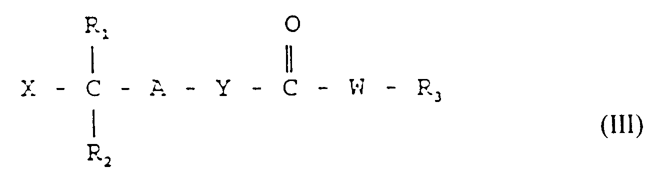
dans laquelle X est un groupe partant autre que Cl;
R₁ et R₂ sont identiques ou différents et sont choisis dans le groupe constitué par un atome d'hydrogène et les groupes alkyle saturés ou insaturés, linéaires ou ramifiés, aryle et benzyle;
A est un noyau phényle substitué ou non substitué dans lequel les substituants sont choisis dans le groupe constitué par les groupes aryle, alkyle, halogéno, nitro, alcoxy et leurs combinaisons;
Y est une chaîne hydrocarbonée possédant un groupe alkyle saturé ou insaturé, linéaire ou ramifié, aryle ou aralkyle qui peut être lié à A par une liaison éther;
W est un atome de soufre ou d'oxygène; et
R₃ est un groupe dérivé d'un alcool, d'un phénol, d'un thiol, d'un thiophénol ou d'un résidu hétérocyclique.

2. Composé selon la revendication 1, dans lequel
R₃ est choisi dans le groupe constitué par les groupes dichlorophényle, pentafluorophényle, pentachlorophényle, 2-ninophényle, 2,4-dinitrophényle, 4-nitrophényle, 2-chlorophényle, 2,4,5-trichlorophényle, 2-bromophényle, 4-bromophényle, trichloroéthyle phénacyle et les hétérocycles de 3-12 atomes de carbone contenant un ou plusieurs atomes d'azote, de soufre ou d'oxygène, ou leurs combinaisons;
X est un atome d'halogène; et
Y est -(O)ₘ-(CH₂)ₙ-, où m est nul ou égal à un et n est un nombre entier de plus de zéro à douze.

3. Composé selon la revendication 1. de formule 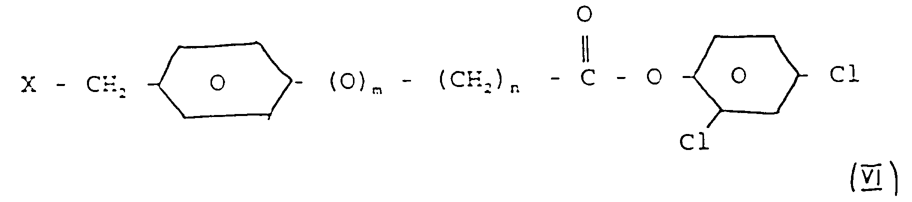 dans laquelle X est un atome d'halogène autre que Ci; m est nul ou égal à un et n est un nombre entier de plus de zéro à 12.

4. Composé énantiomère pur de formule: 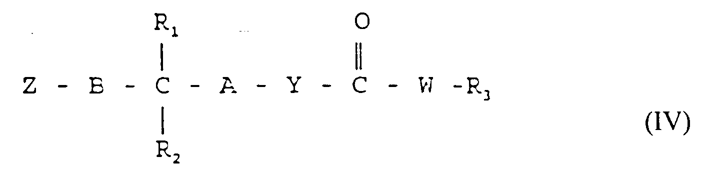
dans laquelle Z est un groupe protecteur d'acide N-α-aminé autre qu'un groupe dithiosuccinyle ou (isopropyldithio)carbonyle;
B est un acide aminé D ou L existant à l'état naturel possédant des chaînes latérales protégées ou non protégées;
R₁ et R₂ sont identiques ou différents et sont choisis dans le groupe constitué par un atome d'hydrogène et les groupes alkyle saturés ou insaturés, linéaires ou ramifiés, aryle et benzyle;
A est un noyau phényle substitué ou non substitué dans lequel les substituants sont choisis dans le groupe constitué par les groupes aryle, alkyle, halogéno, nitro, alcoxy et leurs combinaisons;
Y est une chaîne hydrocarbonée possédant un groupe alkyle saturé ou insaturé, linéaire ou ramifié, aryle ou aralkyle qui peut être lié à A par une liaison éther;
W est un atome de soufre ou d'oxygène; et
R₃ est un groupe dérivé d'un alcool, d'un phénol, d'un thiol d'un thiophénol ou d'un résidu hétérocyclique.

5. Composé selon la revendication 4, dans lequel
R₃ est choisi dans le groupe constitué par les groupes dichlorophényle. pentafluorophényle, pentachlorophényle 2-nitrophényle, 2,4-dinitrophényle, 4-nitrophényle. 2-chlorophényle 2-bromophényle, 4-bromophényle, trichloroéthyle, phénacyle et les hétérocycles de 3-12 atomes de carbone contenant un ou plusieurs atomes d'azote. de soufre ou d'oxygène. ou leurs combinaisons; et
Y est -(O)ₘ-(CH₂)ₙ-. où m est nul ou égal à un et n est un nombre entier de plus de zéro à douze.

6. Composé selon la revendication 4, dans lequel le groupe protecteur d'acide N-α-aminé et le groupe protecteur des chaînes latérales d'acide aminé sont choisis indépendamment dans le groupe constitué par les groupes 9-fluorénylméthyloxycarbonyle, t-butyloxycarbonyle, t-butyle, trityle, 4-méthoxy-2,3,6-triméthylbenzènesulfonyle, 4,4'-diméthoxybenzhydryle, thio-t-butylacétamidométhyle, carbobenzyloxycarbonyle, 2-nitropyridinesulfényle, trifluoroacétyle, 2-(p-biphénylisopropyloxycarbonyle), 4-méthoxybenzyloxycarbonyle, tosyle, 9-phénylfluorényle, 2-nitrophénylsulfényle, benzyle, p-nitrocarbobenzyloxycarbonyle, t-amyloxycarbonyle, 2,2-[bis(4-nitrophényl)]éthyloxycarbonyle, adamantyloxycarbonyle, 2,2,2-trichloroéthyloxycarbonyle, dinitrophényle, t-butyloxyméthyle, benzyloxyméthyle, nichloroéthyle, nitrobenzyle, phénacyle, 2,2,5,7,8-pentaméthylchromane-6-sulfonyle, benzhydrylamine, méthylbenzhydrylamine, xanthyle et 2,4,6-triméthoxybenzyle.

7. Composé selon la revendication 4, de formule: 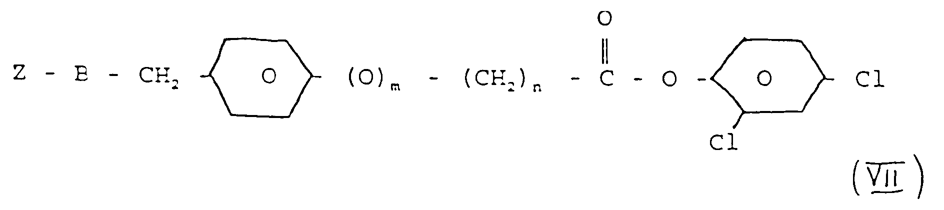
dans laquelle Z est un groupe protecteur d'acide N-α-aminé autre qu'un coupe dithiosuccinyle ou (isopropyldithio)carbonyle;
B est un acide aminé D ou L existant à l'état naturel possédant des chaînes latérales protégées ou non protégées; et
m est nul ou égal à un et n est un nombre entier de plus de zéro à 12.

8. Composé selon la revendication 7, dans lequel le groupe protecteur d'acide N-α-aminé et le groupe protecteur des chaînes latérales d'acide aminé sont choisis indépendamment dans le groupe constitué par les groupes 9-fluorénylméthyloxycarbonyle, t-butyloxycarbonyle. t-butyle. trityle, thio-t-butyle, 4-méthoxy-2,3,6-triméthylbenzènesulfonyle, 4,4-diméthoxybenzhydryle, acétamidométhyle carbobenzyloxycarbonyle, 2-nitropyridinesulfényle, trifluoroacétyle 2-(p-biphénylisopropyloxycarbonyle), 4-méthoxybenzyloxycarbonyle, tosyle, 9-phénylfluorényle, 2-nitrophénylsulfényle. benzyle, p-nitrocarbobenzyloxycarbonyle, t-amyloxycarbonyle, 2,2-[bis(4-nitrophényl)]éthyloxycarbonyle, adamantyloxycarbonyle, 2,2,2-trichloroéthyloxycarbonyle, dinitrophényle, t-butyloxyméthyle, benzyloxyméthyle, trichloroéthyle, nitrobenzyle, phénacyle, 2,2,5,7,8-pentaméthylchromane-6-sulfonyle, benzydrylamine, méthylbenzhydrylamine, xanthyle et 2,4,6-triméthoxybenzyle.

9. Composé de formule: 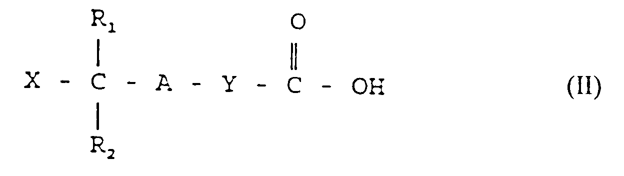
dans laquelle X est un groupe partant autre que Cl;
R₁ et R₂ sont identiques ou différents et sont choisis dans le groupe constitué par un atome d'hydrogène et les groupes alkyle, aryle et benzyle;
A est un noyau phényle substitué ou non substitué dans lequel les substituants sont choisis dans le groupe constitué par les groupes aryle, alkyle, halogéno, nitro, alcoxy et leurs combinaisons; et
Y est une chaîne hydrocarbonée possédant un groupe saturé ou insaturé, linéaire ou ramifié, aryle ou aralkyle qui peut être lié à A par une liaison éther.

10. Composé selon la revendication 9, dans lequel
X est un atome d'halogène autre que Cl; et
Y est -(O)ₘ-(CH₂)ₙ-, où m est nul ou égal à un et n est un nombre entier de plus de zéro à douze.

11. Composé selon la revendication 9, de formule 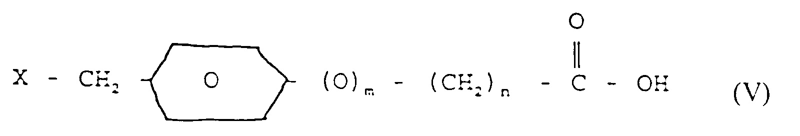 dans laquelle X est un atome d'halogène autre que Cl; m est nul ou égal à un et n est un nombre entier de plus de zéro à 12.

12. Composé selon la revendication 11, dans lequel la formule est l'acide 4-bromométhylphénoxyacétique ou l'acide 4-iodométhylphénoxyacétique.

13. Procédé de préparation d'un composé de formule: 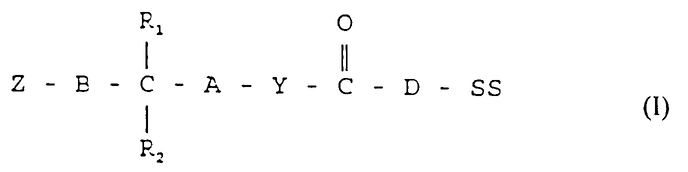
dans laquelle dans laquelle Z est un groupe protecteur d'acide N-α-aminé autre qu'un groupe dithiosuccinyle ou (isopropyldithio)carbonyle;
B est un acide aminé D ou L, existant à l'état naturel possédant des chaînes latérales protégées ou non protégées;
R₁ et R₂ sont identiques ou différents et sont choisis dans le groupe constitué par un atome d'hydrogène et les groupes alkyle saturés ou insaturés, linéaires ou ramifiés, aryle et benzyle;
A est un noyau phényle substitué ou non substitué dans lequel les substituants sont choisis dans le groupe constitué par les groupes aryle, alkyle, halogéno, nitro, alcoxy et leurs combinaisons;
Y est une chaîne hydrocarbonée possédant un groupe alkyle saturé ou insaturé, linéaire ou ramifié, aryle ou aralkyle qui peut être lié à A par une liaison éther;
D est un atome d'oxygène ou un groupe -NH- qui peut être lié à un groupe espaceur choisi dans le groupe constitué par un acide aminé naturel ou non naturel et une chaîne hydrocarbonée possédant un groupe alkyle saturé ou insaturé, linéaire ou ramifié, aryle ou aralkyle, le groupe espaceur étant lié au groupe carbonyle par un atome d'oxygène ou une liaison -NH-; et
SS est un support solide qui est lié au groupe carbonyle par l'intermédiaire de D et qui est choisi dans le groupe constitué par le polystyrène réticulé avec du divinylbenzène, le polydiméthylacrylamide réticulé, le verre, le verre à porosité contrôlée, le poly(difluorure de vinylidène), le polypropylène, le polyéthylène. les céramiques. les silices et les alumines; comprenant les étapes consistant à:
a. fournir un composé de formule: 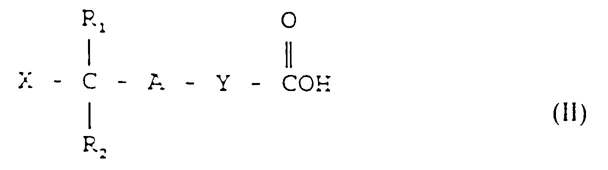 dans laquelle X est un groupe partant autre que Cl et A, R₁, R₂ et Y sont définis ci-dessus;
b. faire réagir le composé de l'étape (a) avec un alcool, un phénol, un thiol où un thiophénol, en présence d'un agent de condensation, pour former un produit de condensation de formule: 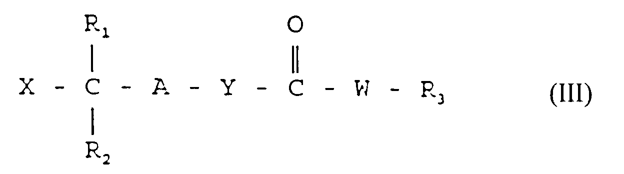
dans laquelle X, R₁, R₂ et Y sont définis ci-dessus;
W est un atome de soufre ou d'oxygène; et
R₃ est un groupe dérivé d'un alcool, d'un phénol, d'un thiol, d'un thiophénol ou d'un alcool ou thiol hétérocyclique analogue;
c. faire réagir le produit de l'étape (b) avec un acide aminé protégé dans des conditions suffisantes pour déplacer X et lier l'acide aminé au composé par une liaison ester; et
d. faire réagir le produit de l'étape (c) avec un support solide fonctionnalisé dans des conditions appropriées pour éliminer WR₃ et le coupler avec le support solide fonctionnalisé.

14. Procédé selon la revendication 13, dans lequel l'alcool, le phénol, le thiol ou le thiophénol est choisi dans le groupe constitué par les phénols substitués, le 2,4-dichlorophénol le trichloroéthanol, l'alcool phénacylique, le N-hydroxysuccinimide, les hétérocycles hydroxylés et les thiols substitués.

15. Procédé selon la revendication 13, dans lequel le composé préparé a pour formule; 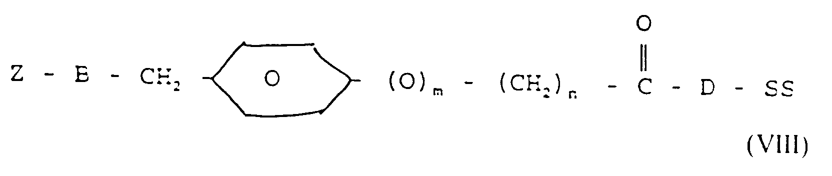
dans laquelle dans laquelle Z est un groupe protecteur d'acide N-α-aminé autre qu'un groupe dithiosuccinyle ou (isopropyldithio)carbonyle;
B est un acide aminé D ou L existant à l'état naturel possédant une chaîne latérale protégée ou non protégée;
D est un atome d'oxygène ou un groupe -NH- qui peut être lié à un groupe espaceur choisi dans le groupe constitué par un acide aminé naturel ou non naturel et une chaîne hydrocarbonée possédant un groupe alkyle saturé ou insaturé, linéaire ou ramifié, aryle ou aralkyle, le groupe espaceur étant lié au groupe carbonyle par un atome d'oxygène ou une liaison -NH-; et
SS est un support solide choisi dans le groupe constitué par le polystyrène réticulé avec du divinylbenzène, le polydiméthylacrylamide réticulé, le verre, le verte à porosité contrôlée, le poly(difluorure de vinylidène), le polypropylène, le polyéthylène, les céramiques, les silices et les alumines, qui est lié au groupe carbonyle par l'intermédiaire de D;
dans laquelle m est nul ou égal à un et n est un nombre entier de plus de zéro à 12.

16. Procédé de préparation d'un composé de formule: 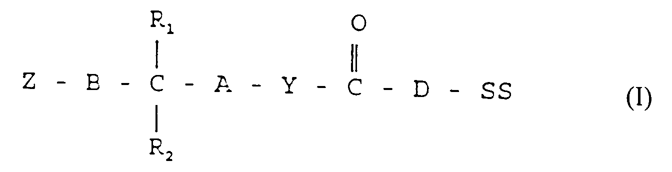
dans laquelle dans laquelle Z est un groupe protecteur d'acide N-α-aminé autre qu'un coupe dithiosuccinyle ou (isopropyldithio)carbonyle;
B est un acide aminé D ou L existant à l'état naturel possédant des chaînes latérales protégées ou non protégées;
R₁ et R₂ sont identiques ou différents et sont choisis dans le groupe constitué par un atome d'hydrogène et un groupe alkyle saturé ou insaturé. linéaire ou ramifié, aryle et benzyle;
A est un noyau phényle substitué ou non substitué dans lequel les substituants sont choisis dans le groupe constitué par les groupes aryle, alkyle, halogéno. nitro, alcoxy et leurs combinaisons:
Y est une chaîne hydrocarbonée possédant un groupe alkyle saturé ou insaturé, linéaire ou ramifié, aryle ou aralkyle qui peut être lié à A par une liaison éther;
D est un atome d'oxygène ou un groupe -NH- qui peut être lié à un groupe espaceur choisi dans le groupe constitué par un ou plusieurs acides aminés naturels ou non naturels et une chaîne hydrocarbonée possédant un groupe alkyle saturé ou insaturé, linéaire ou ramifié, aryle ou aralkyle, le groupe espaceur étant lié au groupe carbonyle par un atome d'oxygène ou une liaison -NH-; et
SS est un support solide qui est lié au groupe carbonyle par l'intermédiaire de D et qui est choisi dans le groupe constitué par le polystyrène réticulé avec du divinylbenzène, le polydiméthylacrylamide réticulé, le verre, le verre à porosité contrôlée, le poly(difluorure de vinylidène), le polypropylène, le polyéthylène, les céramiques, les silices et les alumines; comprenant les étapes consistant à:
a. fournir un précurseur acide carboxylique aromatique de formule: 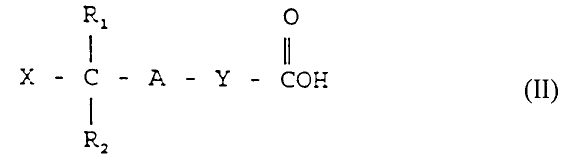 dans laquelle X est un groupe partant autre que Cl et R₁, R₂, A et Y sont définis ci-dessus;
b. faire réagir le composé de l'étape (a) avec une source d'hydroxyde dans des conditions suffisantes pour remplacer X par un groupe hydroxyle;
c. condenser un support solide fonctionnalisé avec le produit de l'étape (b):
d. faire réagir le produit de l'étape (c) avec un réactif dans des conditions suffisantes pour remplacer le groupe hydroxyle par un groupe partant; et
e. faire réagir le produit de l'étape (d) avec un acide aminé protégé autre qu'un sel de césium d'acide aminé protégé dans des conditions suffisantes pour déplacer le groupe partant et coupler l'acide aminé au composé par une liaison ester de manière à ne pas avoir de racémisation.

17. Procédé selon la revendication 16 dans lequel le composé préparé a pour formule: 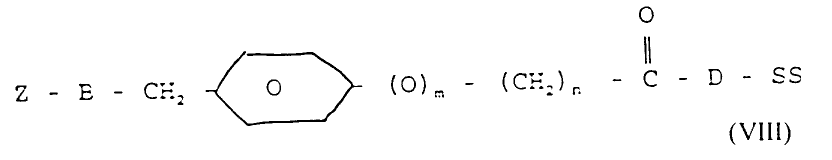
dans laquelle dans laquelle Z est un groupe protecteur d'acide N-α-aminé autre qu'un coupe dithiosuccinyle ou (isopropyldithio)carbonyle;
B est un acide aminé D ou L existant à l'état naturel possédant une chaîne latérale protégée ou non protégée;
D est un atome d'oxygène ou un groupe -NH- qui peut être lié à un groupe espaceur choisi dans le groupe constitué par un ou plusieurs acides aminés naturels ou non naturels et une chaîne hydrocarbonée possédant un groupe alkyle saturé ou insaturé, linéaire ou ramifié, aryle ou aralkyle, le groupe espaceur étant lié au groupe carbonyle par un atome d'oxygène ou une liaison -NH-; et
SS est un support solide qui est lié au groupe carbonyle par l'intermédiaire de D;
dans laquelle m est nul ou égal à un et n est un nombre entier de plus de zéro à 12.

18. Procédé de préparation d'un composé de formule: 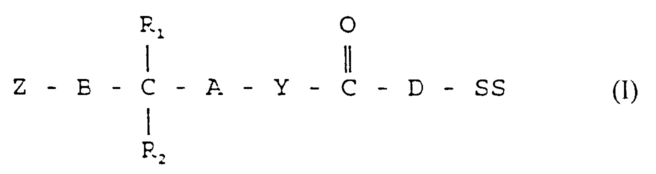
dans laquelle dans laquelle Z est un groupe protecteur d'acide N-α-aminé autre qu'un coupe dithiosuccinyle ou (isopropyldithio)carbonyle;
B est un acide aminé D ou L existant à l'état naturel possédant des chaînes latérales protégées ou non protégées;
R₁ et R₂ sont identiques ou différents et sont choisis dans le groupe constitué par un atome d'hydrogène et les groupes alkyle saturés ou insaturés, linéaires ou ramifiés, aryle et benzyle;
A est un noyau phényle substitué ou non substitué dans lequel les substituants sont choisis dans le groupe constitue. par les groupes aryle. alkyle, halogéno. nitro, alcoxy et leurs combinaisons:
Y est une chaîne hydrocarbonée possédant un groupe alkyle saturé ou insaturé, linéaire ou ramifié, aryle ou aralkyle qui peut être lié à A par une liaison éther;
D est un atome d'oxygène ou un groupe -NH- qui peut être lié à un groupe espaceur choisi dans le groupe constitué par un ou plusieurs acides aminés naturels ou non naturels et une chaîne hydrocarbonée possédant un groupe alkyle saturé ou insaturé, linéaire ou ramifié, aryle ou aralkyle, le groupe espaceur étant lié au groupe carbonyle par un atome d'oxygène ou une liaison -NH-; et
SS est un support solide qui est lié au groupe carbonyle par l'intermédiaire de D et qui est choisi dans le groupe constitué par le polystyrène réticulé avec du divinylbenzène, le polydiméthylacrylamide réticulé, le verte, le verte à porosité contrôlée, le poly(difluorure de vinylidène), le polypropylène, le polyéthylène, les céramiques, les silices et les alumines; comprenant les étapes consistant à:
a. fournir un composé lié à un support solide de formule: 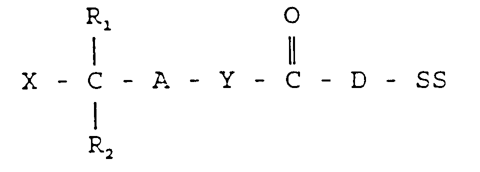 dans laquelle X est un groupe partant autre que Cl et R₁, R₂, A, Y, D et SS sont définis ci-dessus; et
b. faire réagir le produit de l'étape (a) avec un acide aminé protégé autre qu'un sel de césium d'acide aminé protégé dans des conditions suffisantes pour déplacer le groupe partant et coupler l'acide aminé au composé par une liaison ester de manière à ne pas avoir de racémisation.

19. Procédé de synthèse d'un peptide. comprenant les étapes consistant à:
a. fournir un acide aminé lié à un support solide de formule: 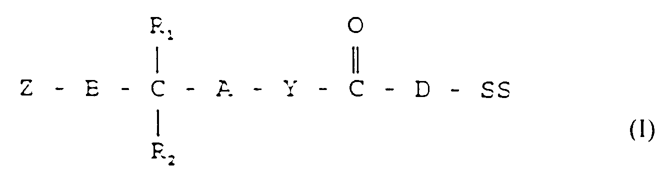
dans laquelle dans laquelle Z est un groupe protecteur d'acide N-α-aminé autre qu'un groupe dithiosuccinyle ou (isopropyldithio)carbonyle;
B est un acide aminé D ou L existant à l'état naturel possédant des chaînes latérales protégées ou non protégées;
R₁ et R₂ sont identiques ou différents et sont choisis dans le groupe constitué par un atome d'hydrogène et les groupes alkyle saturés ou insaturés, linéaires ou ramifiés, aryle et benzyle;
A est un noyau phényle substitué ou non substitué dans lequel les substituants sont choisis dans le groupe constitué par les groupes aryle, alkyle, halogéno, nitro, alcoxy et leurs combinaisons;
Y est une chaîne hydrocarbonée possédant un groupe alkyle saturé ou insaturé, linéaire ou ramifié, aryle ou aralkyle qui peut être lié à A par une liaison éther;
D est un atome d'oxygène ou un groupe -NH- qui peut être lié à un groupe espaceur choisi dans le groupe constitué par un ou plusieurs acides aminés naturels ou non naturels et une chaîne hydrocarbonée possédant un groupe alkyle saturé ou insaturé, linéaire ou ramifié, aryle ou aralkyle, le groupe espaceur étant lié au groupe carbonyle par un atome d'oxygène ou une liaison -NH-; et
SS est un support solide qui est lié au groupe carbonyle par l'intermédiaire de D et qui est choisi dans le groupe constitué par le polystyrène réticulé avec du divinylbenzène. le polydiméthylacrylamide réticulé, le verre, le verre à porosité contrôlée, le poly(difluorure de vinylidène), le polypropylène, le polyéthylène. les céramiques. les silices et les alumines: et
b. coupler successivement les acides aminés protégés. autres que les sels de césium d'acide aminé protégé, avec B pour produire un peptide lié à un support solide.

20. Procédé selon la revendication 19, comprenant. en outre. l'étape consistant à éliminer les groupes protecteurs du peptide lié à un support solide.

21. Procédé selon la revendication 19, dans lequel le peptide synthétisé est coupé du support solide.

22. Procédé selon la revendication 19, dans lequel l'acide aminé lié à un support solide répond à la formule: 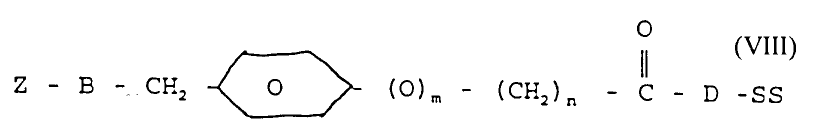
dans laquelle dans laquelle Z est un groupe protecteur d'acide N-α-aminé autre qu'un groupe dithiosuccinyle ou (isopropyldithio)carbonyle;
B est un acide aminé D ou L existant à l'état naturel possédant une chaîne latérale protégée ou non protégée;
m est nul ou égal à un et n est un nombre entier de plus de zéro à 12;
D est un atome d'oxygène ou un groupe -NH- qui peut être lié à un groupe espaceur choisi dans le groupe constitué par un acide aminé naturel ou non naturel et une chaîne hydrocarbonée possédant un groupe alkyle saturé ou insaturé, linéaire ou ramifié, aryle ou aralkyle, le groupe espaceur étant lié au groupe carbonyle par un atome d'oxygène ou une liaison -NH-; et
SS est un support solide qui est lié au groupe carbonyle par l'intermédiaire de D et qui est choisi dans le groupe constitué par le polystyrène réticulé avec du divinylbenzène, le polydiméthylacrylamide réticulé, le verre, le verre à porosité contrôlée, le poly(difluorure de vinylidène), le polypropylène, le polyéthylène, les céramiques, les silices et les alumines.

23. Procédé de préparation d'un composé selon la revendication 19, comprenant les étapes consistant à:
a. fournir un composé possédant un atome d'hydrogène benzylique de formule: 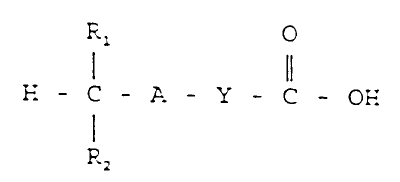
dans laquelle R₁ et R₂ sont identiques ou différents et sont choisis dans le groupe constitué par un atome d'hydrogène et les groupes alkyle saturés ou insaturés, linéaires ou ramifiés, aryle et benzyle:
A est un noyau phényle substitué ou non substitué dans lequel les substituants sont choisis dans le groupe constitué par les groupes aryle, alkyle, halogéno, nitro, alcoxy et leurs combinaisons:
Y est une chaîne hydrocarbonée possédant un groupe alkyle saturé ou insaturé, linéaire ou ramifié, aryle ou aralkyle qui peut être lié à A par une liaison éther; et
b. faire réagir le composé de l'étape (a) avec un réactif, dans des conditions appropriées pour remplacer l'atome d'hydrogène benzylique par le groupe partant.

24. Procédé de préparation d'un composé selon la revendication 1, comprenant les étapes consistant à:
a. fournir un composé de formule 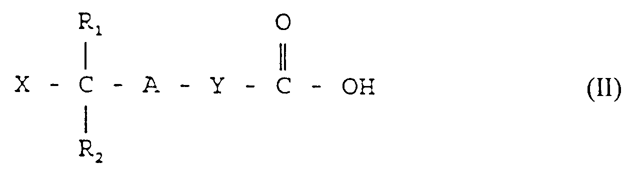
dans laquelle X est un groupe partant autre que Cl;
R₁ et R₂ sont identiques ou différents et sont choisis dans le groupe constitué par un atome d'hydrogène et les groupes alkyle saturés ou insaturés, linéaires ou ramifiés, aryle et benzyle;
A est un noyau phényle substitué ou non substitué dans lequel les substituants sont choisis dans le groupe constitué par les groupes aryle, alkyle, halogéno, nitro, alcoxy et leurs combinaisons;
Y est une chaîne hydrocarbonée possédant un groupe alkyle saturé ou insaturé linéaire ou ramifié, aryle ou aralkyle qui peut être lié à A par une liaison éther; et
b. faire réagir le composé de l'étape (a) avec un alcool, un phénol, un thiol ou un thiophénol, qui peut être hétérocyclique, en présence d'un agent de condensation, dans des conditions appropriées pour produire le composé de la revendication 1 ci-dessus, dans lequel WR₃ est un groupe analogue à l'alcool, au phénol, au thiol ou au thiophénol.

25. Procédé selon la revendication 24, dans lequel l'alcool, le phénol, le thiol ou le thiophénol est chioisi dans le groupe constitué par les phénols substitués, le 2,4-dichlorophénol, le trichloroéthanol, l'alcool phénacylique, le N-hydroxysuccinimide, les hétérocycles hydroxylés et les thiols substitués.

26. Procédé selon la revendication 25, comprenant, en outre. l'étape consistant à:
c. remplacer X par un acide aminé protégé dans des conditions appropriées pour lier l'acide aminé au composé par une liaison ester.

27. Procédé de préparation d'un composé de formule: 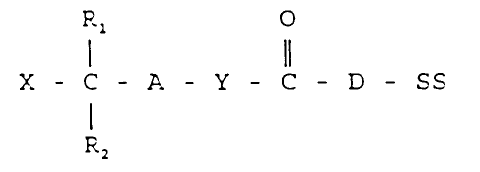
dans laquelle X est un groupe partant autre que Cl;
R₁ et R₂ sont identiques ou différents et sont choisis dans le groupe constitué par un atome d'hydrogène et les groupes alkyle saturés ou insaturés, linéaires ou ramifiés, aryle et benzyle;
A est un noyau phényle substitué ou non substitué dans lequel les substituants sont choisis dans le groupe constitué par les groupes aryle, alkyle, halogéno, nitro, alcoxy et leurs combinaisons;
Y est une chaîne hydrocarbonée possédant un groupe alkyle saturé ou insaturé, linéaire ou ramifié, aryle ou aralkyle qui peut être lié à A par une liaison éther;
D est un atome d'oxygène ou un groupe -NH- qui peut être lié à un groupe espaceur choisi dans le groupe constitué par un ou plusieurs acides aminés naturels ou non naturels et une chaîne hydrocarbonée possédant un groupe alkyle saturé ou insaturé linéaire ou ramifié, aryle ou aralkyle, le groupe espaceur étant lié au groupe carbonyle par un atome d'oxygène ou une liaison -NH-; et
SS est un support solide qui est lié au groupe carbonyle par l'intermédiaire de D et qui est choisi dans le groupe constitué par le polystyrène réticulé avec du divinylbenzène, le polydiméthylacrylamide réticulé, le verre, le verre à porosité contrôlée, le poly(difluorure de vinylidène), le polypropylène, le polyéthylène, les céramiques, les silices et les alumines;
comprenant les étapes consistant à:
a fournir un composé de formule: 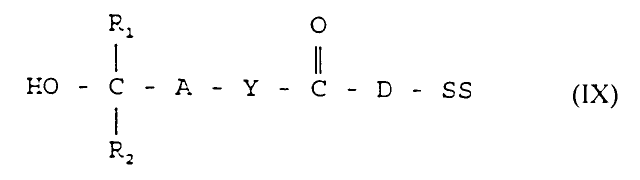 dans laquelle R₁, R₂, A, Y, D et SS sont définis ci-dessus; et
b. faire réagir le composé de l'étape (a) avec un réactif dans des conditions suffisantes pour déplacer le groupe hydroxyle avec un groupe partant X.

28. Procédé selon la revendication 27, dans lequel le composé préparé répond à la formule 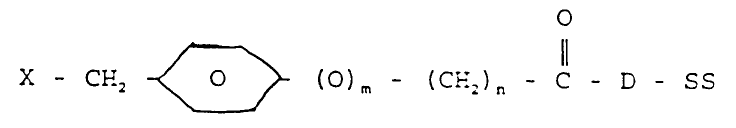
dans laquelle X est un atome d'halogène autre que Cl;
m est nul ou égal à un et n est un nombre entier de plus de zéro à 12.

29. Procédé selon la revendication 27, comprenant, en outre, l'étape consistant à:
c. faire réagir le composé de l'étape (b) avec un acide aminé protégé dans des conditions suffisantes pour déplacer le groupe partant et coupler l'acide aminé au composé par une liaison ester de manière à ne pas avoir de racémisation.

30. Procédé de préparation d'un composé de formule: 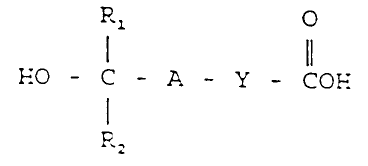
dans laquelle R₁ et R₂ sont identiques ou différents et sont choisis dans le groupe constitué par un atome d'hydrogène et les groupes alkyle saturés ou insaturés, linéaires ou ramifiés aryle et benzyle;
A est un noyau phényle substitué ou non substitué dans lequel les substituants sont choisis dans le groupe constitué par les groupes aryle, alkyle, halogéno, nitro, alcoxy et leurs combinaisons;
Y est une chaîne hydrocarbonée possédant un groupe alkyle saturé ou insaturé, linéaire ou ramifié, aryle ou aralkyle qui peut être lié à A par une liaison éther;
comprenant les étapes consistant à:
a. fournir un composé de formule: 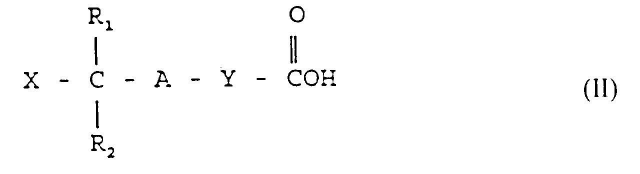 dans laquelle X est un groupe partant autre que Cl et R₁, R₂, A et Y sont définis ci-dessus; et
b. faire réagir le composé de l'étape (a) avec une source d'hydroxyde dans des conditions suffisantes pour remplacer X par un groupe hydroxyle.

31. Procédé selon la revendication 30, dans lequel le composé de l'étape (a) est l'acide 4-bromométhylphénoxyacétique.
